(19) Europäisches Patentamt
European Patent Office
Office européen des brevets

(11) **EP 1 909 189 A2**

(12) **EUROPÄISCHE PATENTANMELDUNG**

(43) Veröffentlichungstag:
**09.04.2008 Patentblatt 2008/15**

(51) Int Cl.:
***G06F 17/00*** *(2006.01)*

(21) Anmeldenummer: **07003449.1**

(22) Anmeldetag: **19.02.2007**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK RS**

(30) Priorität: **20.02.2006 DE 102006007879**
**11.05.2006 DE 102006022056**

(71) Anmelder: **Fraunhofer-Gesellschaft zur Förderung der angewandten Forschung e.V.**
**80686 München (DE)**

(72) Erfinder:
• **Tobola, Andreas**
**91052 Erlangen (DE)**
• **Vogl, Ulrich**
**92263 Ebermannsdorf (DE)**
• **Moersdorf, Hans-Joachim**
**90763 Fuerth (DE)**

(74) Vertreter: **Zinkler, Franz et al**
**Schoppe, Zimmermann, Stöckeler & Zinkler**
**Postfach 246**
**82043 Pullach bei München (DE)**

(54) **Spektralanalyse zur zuverlässigieren Bestimmung von Vitalparametern**

(57)     Vorrichtung zum Bestimmen eines Spektralverhältnisses zwischen einem ersten Signal mit einem ersten Spektrum, das von einer biologischen Größe abhängt und einem zweiten Signal mit einem zweiten Spektrum, das von einer biologischen Größe abhängt, wobei das erste Signal und das zweite Signal eine Mehrzahl von Harmonischen eines periodischen Signals haben, mit einem Rechner zur Berechnung eines ersten Wellenverhältnisses zwischen einem Spektralwert des ersten Spektrums, der eine Frequenz einer Harmonischen der Mehrzahl von Harmonischen hat, und einem Spektralwert des zweiten Spektrums, der die Frequenz der Harmonischen hat; und zur Berechnung eines zweiten Wellenverhältnisses zwischen einem Spektralwert des ersten Spektrums, der eine Frequenz einer anderen Harmonischen der Mehrzahl von Harmonischen hat, und einem Spektralwert des zweiten Spektrums, der die Frequenz der anderen Harmonischen hat. Ferner weist die Vorrichtung einen Optimierer auf, zum Ermitteln des Spektralverhältnisses unter Verwendung des ersten und des zweiten Wellenverhältnisses, wobei der Optimierer ausgebildet ist, um das Spektralverhältnis so zu ermitteln, dass es sich von dem ersten und dem zweiten Wellenverhältnis unterscheidet und ein Optimierungskriterium erfüllt.

FIG 1A

EP 1 909 189 A2

**Beschreibung**

Die vorliegende Erfindung bezieht sich auf eine Vorrichtung zum Bestimmen eines Spektralverhältnisses von zwei Spektren zweier zeitdiskreter Signale, zur zuverlässigeren Bestimmung eines Vitalparameters aus dem Spektralverhältnis und den Spektren. Das Verfahren findet Anwendung in Plethysmogramm-basierenden Messverfahren (z. B. Plethysmographie, Pulsoximetrie) zum Zwecke einer niedrigeren Störanfälligkeit bei Umgebungslichtinterferenzen und elektromagnetischen Interferenzen.

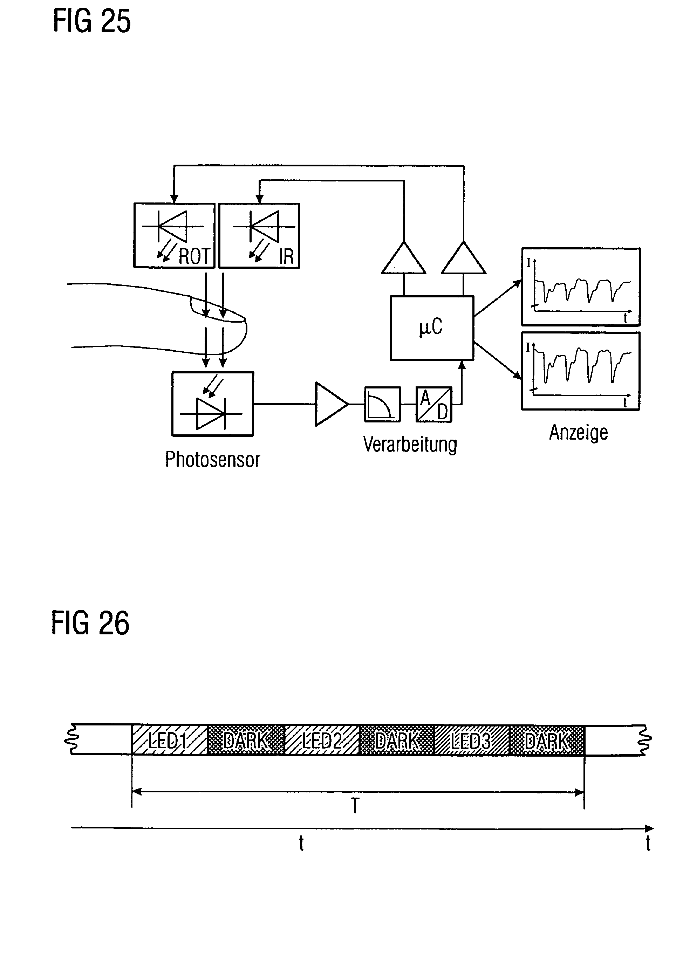

Die Plethysmographie ist ein optisches Verfahren zur Gewinnung eines sogenannten Plethysmogramms, das Auskunft über die Pulsfrequenz und Blutsauerstoffsättigung eines Probanden liefert. Unter einem Plethysmogramm versteht man eine graphische Abbildung von Volumenänderungen. In diesem Anwendungsgebiet werden speziell die Volumenänderungen eines arteriellen Blutstroms an einer örtlich begrenzten Messstelle am menschlichen Körper als das Plethysmogramm aufgenommen. Um dies technisch umzusetzen, wird Gewebe an einer Körperstelle mit arteriellen Blutgefäßen mit Licht durchstrahlt. Dem Patienten wird ein Sensor angelegt, der eine Lichtquelle und einen Photoempfänger enthält, so dass das Licht die Gewebeschicht passiert und die verbleibende Lichtintensität auf den Photoempfänger trifft. Das Licht erfährt im Inneren des Körpers eine Dämpfung, die unter anderem abhängig von der Wellenlänge der Lichtquelle, der Art und der Konzentration der Stoffe im durchstrahlten Gewebe und der Pulsation des Blutes ist. Das so gewonnene Signal des Photoempfängers liegt in Form eines Photostroms vor, ist von den oben genannten Rahmenbedingungen abhängig und entspricht in erster Näherung den durch Herzmuskelkontraktion verursachten Blutvolumenänderungen arterieller Gefäße. Fig. 25 zeigt den prinzipiellen Aufbau einer Vorrichtung zur Erfassung eines Plethysmogramms. Ein Mikrokontroller ($\mu$C) steuert dabei über zwei Treiberstufen zwei LEDs unterschiedlicher Wellenlängen an, prinzipiell genügt zur Erstellung eines Plethysmogramms auch eine Lichtquelle. Die in Fig. 25 dargestellten LEDs emittieren Licht im Rot- und Infrarotbereich. Das von den LEDs emittierte Licht passiert dann das Gewebe des Probanden, in Fig. 25 ist dies exemplarisch als Finger dargestellt. Nachdem das Licht das Gewebe des Probanden passiert hat, trifft es auf einen Photosensor. Der Photosensor wandelt die optischen Signale in elektrische Signale und gibt diese an eine Verarbeitungselektronik weiter, die das Signal verstärkt, analog-digital wandelt und dem Mikrokontroller ($\mu$C) zuführt. Der Mikrokontroller ($\mu$C) ermittelt dann aus den ihm zugeführten Digitalsignalen zwei Plethysmogramme, je ein Plethysmogramm pro Wellenlänge. Aus den Signalverläufen der so gemessenen Plethysmogramme, lassen sich Vitalparameter, wie z.B. die Herzfrequenz oder die Blutsauerstoffsättigung des Probanden bestimmen, wobei zur Bestimmung der Herzfrequenz auch prinzipiell ein einzelnes Plethysmogramm genügen würde, zur Bestimmung der Blutsauerstoffsättigung sind zwei Plethysmogramme von Lichtquellen unterschiedlicher Wellenlängen notwendig.

Die Pulsoximetrie ist ein nichtinvasives Verfahren zur Messung der Blutsauerstoffsättigung ($SpO_2$) und der Herzfrequenz (HR) mittels eines optischen Sensors. Die durch das Pulsoximeter erfasste Sauerstoffsättigung wird speziell $SpO_2$-Wert genannt. Die Sauerstoffsättigung ist als das Verhältnis aus der Konzentration von sauerstoffgesättigten Hämoglobinmolekülen und der gesamten Hämoglobinkonzentration definiert und wird in Prozent angegeben. Eine Komponente des Pulsoximeters ist ein Sensor mit zwei integrierten Lichtquellen, der ähnlich wie der eines Plethysmographen beschaffen ist, vgl. Fig. 25. In der Pulsoximetrie wird von mindestens zwei Plethysmogrammen Gebrauch gemacht, um die Farbe des arteriellen Blutes zu bestimmen. Die Farbe des Blutes ist wiederum von der Sauerstoffsättigung abhängig. Mit einer geschickten Wahl der Wellenlängen der Lichtquellen lässt sich zeigen, dass aus den Verhältnissen markanter Punkte im Plethysmogramm, eine Größe gewonnen werden kann, die mit der Sauerstoffsättigung gut korreliert. Typischerweise werden die Spektren der Empfangssignale zweier Lichtquellen unterschiedlicher Wellenlänge bestimmt und der Quotient bestimmter Spektralwerte gebildet. Dieser Quotient ist dann näherungsweise proportional zum $SpO_2$-Wert des Blutes.

Ein wesentliches Qualitätsmerkmal beim Vergleich von Pulsoximetern ist die Resistenz gegenüber Störungen. Als besonders problematisch stellt sich die Filterung derjenigen unerwünschten Signalanteile dar, die durch die Bewegung des Patienten entstehen. Schon bei kleinen Bewegungen können die Amplituden der sogenannten Bewegungsartefakte größer als die der Pulswelle im Signal wirken. Ist das Signal stark mit Bewegungsartefakten überlagert, führt das zum vorübergehenden Funktionsausfall der Geräte mit entsprechender Signalisierung dieses Problems. Im schlimmsten Fall detektieren die Geräte die verfälschte Messung nicht und geben kein Signal ab, so dass die angezeigten Messwerte fälschlicherweise für wahr gehalten werden. Die Behandlungsqualität eines Patienten kann sich aufgrund falsch angezeigter Messwerte deutlich verringern. Gerade im Umfeld von Operationssälen stellen die oben genannten Verfälschungen einen großen Nachteil von Pulsoximetern dar.

Neben den Bewegungsartefakten können starke Lichtquellen, wie die von OP-Lampen, Leuchtstoffröhren oder Bildschirmen, zu unerwünschten Interferenzen im Signal führen. Bei herkömmlichen Pulsoximetern bzw. Plethysmographen wird dieses Problem üblicherweise durch Einfügen von zusätzlichen Messperioden zur Umgebungslichtbestimmung und anschließender Subtraktion der Umgebungslichtmessung von der Nutzsignalmessung vermindert. Während dieser Messperioden oder Zeitschlitze, werden alle Lichtquellen des Sensors ausgeschaltet und nur das Umgebungslicht gemessen. Die Umgebungslichtintensität wird von dem Plethysmogramm subtrahiert und damit der Umgebungslichtanteil weitgehend vom Pulssignal getrennt. Dennoch verbleibt gerade bei pulsierenden oder wechselstrombetriebenen Umgebungslichtquellen ein Störanteil im Plethysmogramm. Der Störanteil im Plethysmogramm hängt also stark von den in der Umgebung verwendeten elektronischen Geräten bzw. Störern ab. Gerade in der intensivmedizinischen Versorgung von Patienten, kommt eine Vielzahl elektronischer Geräte und Hilfsmittel zum Einsatz, so dass die Störanfälligkeit von Pulsoximetern und Plethysmographen in intensivmedizinischen Umgebungen besonders gegeben ist. Gerade im Bereich der intensivmedizinischen Versorgung hingegen, sind Messfehler von Vitalparametern wie z.B. der Herzfrequenz oder der Blutsauerstoffsättigung äußerst kritisch und können schwerwiegende Konsequenzen nach sich ziehen.

In der Pulsoximetrie verfügen Transmissions- und Remissionssensoren über mehrere LEDs (Sender) und nur eine Photodiode (Empfänger). Das Gewebe des Probanden wird dabei von LEDs verschiedener Wellenlängen durchleuchtet und die Photodiode empfängt das Licht verschiedener Wellenlängen aus dem Gewebe. Prinzipiell wäre es möglich verschiedene Kanäle anhand der Wellenlängen der LEDs zu unterscheiden, z.B. durch Farbfilter an mehreren Photodioden. Da dies auf der Seite der Photodiode technisch aufwendig ist, müssen die Intensitäten der LEDs moduliert werden. Nur dann ist eine Unterscheidung der Wellenlängen mittels einer einzigen breitbandigen Photodiode möglich.

Um dem Empfänger zu ermöglichen, verschiedene Sendequellen (LEDs) mit verschiedenen Wellenlängen zu unterscheiden, werden bei bekannten Pulsoximetern TDMA-Konzepte (Time Division Multiple Access), also Zeitmultiplexverfahren eingesetzt. Dabei wird jeder Sensor-LED ein Zeitfenster zugewiesen, in dem diese eingeschaltet wird. Fig. 26 illustriert diese zeitliche Abfolge von Signalen. Es ist zu erkennen, dass den verschiedenen LEDs nacheinander Zeitschlitze gleicher Dauer zugeordnet sind, die durch Dunkelperioden gleicher Dauer getrennt sind. Fig. 26 zeigt eine schematische Abfolge mit drei verschiedenen LEDs. Nacheinander leuchten die LEDs verschiedener Wellenlängen, in Fig. 26 sind die Hellzeitdauern der LEDs durch "LED 1", "LED 2" und "LED 3" bezeichnet, für eine kurze Zeitdauer auf. Typische Frequenzen mit denen die Lichtquellen derzeitiger Pulsoximeter angesteuert werden, liegen bei 20-50Hz. Durch Hinzufügen zusätzlicher Dunkelphasen, in denen keine der LEDs leuchtet, in Fig. 26 durch "DARK" bezeichnet, versucht man den durch Umgebungslicht verursachten Signalanteil zu messen und anschließend vom Nutzsignal zu subtrahieren. Dennoch sind die Ergebnisse oftmals durch Umgebungslicht oder Hochfrequenzchirurgieeinflüsse verfälscht. In der Hochfrequenzchirurgie wird Gewebe mittels hochfrequenter Spannungen geschnitten. Diese hohen Frequenzen verursachen Induktionen in Leitungen der Pulsoximeter und können so deren Funktion stören. Die örtlichen Einflüsse können weitgehend unterdrückt werden, da die Sensoren gegen Einstrahlung von außen geschützt sind. Dennoch tritt Umgebungslicht in die Hülle des Sensors ein.

Ein weiteres Beispiel dynamischer Störungen ist bei Probanden zu beobachten, an denen Dauermessungen durchgeführt werden. Diese tragen einen Sensor mit integrierten LEDs und Photoempfänger über einen längeren Zeitraum zur Erfassung von Langzeitdaten. Bei diesen Patienten oder Probanden kommt es nun, beispielsweise bei Autofahrten durch Alleen oder auch Häuserschluchten, zu stark und gegebenenfalls auch schnell wechselnden Lichtverhältnissen. Diese wechselnden Lichtverhältnisse äußern sich stellenweise sehr ähnlich wie die Störungen in stationären Umfeldern von Kliniken. Prinzipiell sind Probanden, die sich in einer Dauermessung befinden, einer Vielzahl von Umgebungslichteinflüssen ausgesetzt, die ein ganzes Spektrum an Störungen hervorrufen können.

Die Subtraktion des Umgebungslichtanteils, ermittelt durch Hinzufügen von Dunkelphasen, verbessert die Signalqualität deutlich. Allerdings verbleiben Störartefakte, die zu falschen $Sp0_2$-Werten führen können. Bislang ist es trotz zahlreicher Versuche nicht möglich, die durch Leuchtstofflampen, Infrarotwärmelampen, Operationsbeleuchtung und Monitore verursachten Störungen aus dem Nutzsignal zu entfernen. Da bei Pulsoximetern und Plethysmographen das Verhältnis zwischen Nutzsignalen, also die Signalanteile, die durch die Volumenänderung des Gewebes hervorgerufen werden, und den Störungen sehr ungünstig sein kann, sind auch Störungen, die durch die Signalverarbeitung weiter verfälscht werden relevant. Beispielsweise werden Signale vor einer Analog/Digitalwandlung mit einem Tiefpass gefiltert, um Fehler durch Unterabtastung zu vermeiden. Da die verwendeten Filter immer nur über eine endliche Dämpfung im Sperrbereich verfügen, entstehen dennoch Fehler durch Unterabtastung oder auch Aliasing-Fehler genannt. Je nach ursprünglicher Störfrequenz werden diese Störungen dann in den Nutzbereich gespiegelt und können dort bei verschiedenen Frequenzen auftreten.

Die Störanfälligkeit derzeitiger Pulsoximeter und Plethysmographen steigt, wenn sich in ihrer Umgebung die oben genannten Störer befinden. Gerade in Operationssälen oder intensivmedizinischen Versorgungsstationen, findet sich eine Vielzahl elektronischer Geräte bzw. elektronischer Störer. Gerade in solchen Umfeldern steigt deswegen die Störanfälligkeit derzeitiger Pulsoximeter und Plethysmographen. Dieser signifikante Nachteil kann ernsthafte Konsequenzen für Probanden nach sich führen, wenn in solchen Situation Messfehler auftreten, die nicht unmittelbar als solche identifiziert werden können.

Bekannte Verfahren zur Plethysmographie sind beispielsweise in folgenden Schriften zu finden:

EP 1374764 A1/WO 2002054950 A08, worin eine prinzipielle Schaltung zur Messung und Erfassung eines Plethysmogramms beschrieben ist und auf die oben beschriebene Signalverarbeitung im Detail eingegangen wird.

EP 208201 A2/A3, worin prinzipiell die optische Erfassung einer Volumenänderung eines Körperteils und ein Auswertegerät zur Auswertung der optischen Signale geschützt wird. Das dort beschriebene Verfahren nutzt dabei die sich verändernde äußerliche Volumenänderung von Extremitäten, die durch den Puls und die damit verbundenen Blutdruckänderungen hervorgerufen wird.

EP 341059 A3. Hier wird ein prinzipielles Verfahren zur Pulsoximetrie beschrieben, das sich Lichtquellen (LEDs) unterschiedlicher Wellenlängen zunutze macht. Dabei wird das Gewebe des Probanden mit Licht unterschiedlicher Wellenlängen durchstrahlt, die Lichtsignale mittels optischen Sensoren aus dem Gewebe aufgenommen und durch eine entsprechende analoge Signalverarbeitung aufgewertet.

EP 314331 B1, ein Verfahren der Pulsoximetrie das ebenfalls auf Licht unterschiedlicher Wellenlängen basiert wird benutzt, um das Gewebe eines Probanden zu durchleuchten. Die so gewonnenen optischen Signale werden in elektrische Signale gewandelt, und aus diesen ein Wert der Aufschluss über die Blutsauerstoffsättigung des Probanden gibt, extrahiert.

EP 1254628 A1, das hier geschützte Pulsoximeter ist ebenfalls ausgelegt eine Blutsauerstoffsättigung zu bestimmen, wobei durch das hier vorgeschlagene Verfahren Störungen durch Nebensprechen zusätzlich vermindert werden.

US 5503144/US 6714803, hier werden Signalverarbeitungsverfahren zur linearen Regression beschrieben, die anhand zweier Plethysmogramme einen $SpO_2$-Wert bestimmen. Dabei wird zwischen den beiden Plethysmogrammen ein Korrelationskoeffizient bestimmt, der als Zuverlässigkeitsmaß dient.

Die Aufgabe der vorliegenden Erfindung ist es, eine Vorrichtung und ein Verfahren zum Bestimmen eines Spektralverhältnisses aus zwei Spektren zweier zeitdiskreter Signale zu schaffen, um einen Vitalparameter, wie beispielsweise eine Herzfrequenz und ein Blutsauerstoffgehalt, zuverlässig zu bestimmen, um eine bessere Behandlungsqualität eines Patienten zu erreichen.

Diese Aufgabe wird gelöst durch eine Vorrichtung gemäß Anspruch 1 und ein Verfahren gemäß Anspruch 28.

Die Aufgabe wird gelöst durch eine Vorrichtung zum Bestimmen eines Spektralverhältnisses zwischen einem ersten Signal mit einem ersten Spektrum, das von einer biologischen Größe abhängt und einem zweiten Signal mit einem zweiten Spektrum, das von der biologischen Größe abhängt, wobei das erste Signal und das zweite Signal eine Mehrzahl von Harmonischen eines periodischen Signals haben. Ferner weist die Vorrichtung einen Rechner zur Berechnung eines ersten Wellenverhältnisses zwischen einem Spektralwert des ersten Spektrums, der eine Frequenz einer der Harmonischen der Mehrzahl von Harmonischen hat, und einem Spektralwert des zweiten Spektrums, der die Frequenz der Harmonischen hat, auf. Ferner dient der Rechner zur Berechnung eines zweiten Wellenverhältnisses zwischen einem Spektralwert des ersten Spektrums, der eine Frequenz einer anderen harmonischen der Mehrzahl von Harmonischen hat, und einem Spektralwert des zweiten Spektrums, der die Frequenz der anderen Harmonischen hat. Die Vorrichtung weist ferner einen Optimierer auf, zum Ermitteln des Spektralverhältnisses und der Verwendung des ersten und des zweiten Wellenverhältnisses, wobei der Optimierer ausgebildet ist, um das Spektralverhältnis so zu ermitteln, dass es sich von dem ersten und dem zweiten Wellenverhältnis unterscheidet und ein Optimierungskriterium erfüllt.

Ferner wird die Aufgabe gelöst durch ein Verfahren zum Bestimmen eines Spektralverhältnisses zwischen einem ersten Signal mit einem ersten Spektrum, das von einer biologischen Größe abhängt und einem zweiten Signal mit einem zweiten Spektrum, das von einer biologischen Größe abhängt, wobei das erste Signal und das zweite Signal eine Mehrzahl von Harmonischen eines periodischen Signals haben, durch Errechnen eines ersten Wellenverhältnisses zwischen einem Spektralwert des ersten Spektrums, der eine Frequenz einer Harmonischen der Mehrzahl von Harmonischen hat, und einem Spektralwert des zweiten Spektrums, der die Frequenz der Harmonischen hat. Ferner umfasst das Verfahren einen Schritt des Berechnens eines zweiten Wellenverhältnisses zwischen einem Spektralwert des ersten Spektrums, der eine Frequenz einer anderen Harmonischen der Mehrzahl von Harmonischen hat, und einem Spektralwert des zweiten Spektrums, der die Frequenz der anderen Harmonischen hat. Das Verfahren umfasst weiterhin einen Schritt des Optimierens zum Ermitteln des Spektralverhältnisses, unter Verwendung des ersten und des zweiten Wellenverhältnisses, wobei das Spektralverhältnis derart bestimmt wird, dass es sich von dem ersten und dem zweiten Wellenverhältnis unterscheidet und ein Optimierungskriterium erfüllt.

Der Kerngedanke der vorliegenden Erfindung ist, die Störanteile, die sich bei der Plethysmographie und Pulsoximetrie dem Nutzsignal überlagern, durch Ausnutzen der Kenntnis über die spektrale Zusammensetzung der Nutzsignale effektiver zu unterdrücken. Zur Bestimmung des Blutsauerstoffgehalts werden bei der Plethysmographie zwei Spektren von Signalen bestimmt, die auf optische Empfangssignale durchleuchteten Gewebes zurückgehen und verschiedene Wellenlängen aufweisen. Da die beiden Spektren von unter Umständen starken Störsignalen überlagert sind, macht man sich bei der vorliegenden Erfindung die Kenntnis über Form und spektrale Zusammensetzung des Pulssignals zu Nutze, bestimmt zunächst diese Frequenzanteile und vollzieht die Bestimmung des Blutsauerstoffgehalts, basierend auf den so bestimmten Frequenzen. Zunächst wird aus den beiden Spektren, die auf den optischen Empfangssignalen verschiedener Wellenlängen basieren, ein Referenzspektrum bestimmt, indem unabhängige Störungen bereits reduziert sind. In diesem Referenzspektrum wird nun nach den Frequenzanteilen eines Pulssignals gesucht, und nachdem diese charakteristischen Frequenzanteile gefunden sind, wird basierend auf diesen ein Linearkoeffizient gebildet, der dann mit erheblich höherer Zuverlässigkeit bestimmt werden kann. Sowohl bei der Bestimmung des Referenzspektrums als auch bei der Bestimmung des Linearkoeffizienten kann von einem mathematischen Verfahren, der sog. Singulärwertzerlegung, Gebrauch gemacht werden, die es erlaubt, das Referenzspektrum und den Linearkoeffizienten mit einer minimalen Ungenauigkeit zu bestimmen.

Ein Ausführungsbeispiel der vorliegenden Erfindung wird nun anhand der Figuren 1 bis 24 im Detail erläutert. Es zeigen:

Fig. 1a)  schematische Darstellung eines erfindungsgemäßen Ausführungsbeispiels;

Fig. 1b)  schematische Darstellung eines erfindungsgemäßen Ausführungsbeispiels;

Fig. 2a)  schematische Darstellung der unregelmäßigen Anordnung der Hellzeitdauern;

Fig. 2b)  regelmäßige Anordnung der Hellzeitdauern gemäß herkömmlichen Pulsoximetern;

Fig. 3  Blockschaltbild einer Realisierung des Ausführungsbeispiels;

Fig. 4  schematisierte Darstellung eines Spektrums eines Signals im Basisband;

Fig. 5  schematisierte Darstellung eines Spektrums eines Signals im Übertragungsband;

Fig. 6  schematische Darstellung zweier orthogonaler Chipfolgen der Länge 101 Chips;

Fig. 7  schematische Darstellung eines Spektrums einer Chipfolge der Länge 101 Chips;

Fig. 8  schematische Darstellung des Signals im 0-bertragungsband;

Fig. 9  schematische Darstellung der Spreizungsstörung und Entspreizung im Frequenzbereich;

Fig. 9a)  schematische Darstellung des Spektrums im Basisband;

Fig. 9b)  schematische Darstellung des Spektrums der Chipfolge;

Fig. 9c)  schematische Darstellung des Spektrums im Übertragungsband;

Fig. 9d)  schematische Darstellung des Spektrums der Nutz- und Störanteile im Basisband nach der Entspreizung;

Fig. 10  zwei beispielhafte empfangene Signalverläufe zweier LEDs unterschiedlicher Wellenlängen;

Fig. 11  Darstellung zweier beispielhafter Signalverläufe für die Dunkel-Dauer bzw. die Umgebungslichtmessung;

Fig. 12  Beispielhafte Übertragungsfunktion eines Extraktionsfilters mit 15 dB Dämpfung und 100 Hz Unterdrückung; Vergrößerung im Bereich von 100 Hz;

Fig. 13  Beispielhafte Signalverläufe der Hellsendekanäle, von denen das Umgebungslichtsignal subtrahiert wurde, die Vergrößerung zeigt das Referenzsignal;

Fig. 14  Schematische Darstellung der Blockbildung zur weiteren Signalverarbeitung, $l_B$ entspricht der Blocklänge, $l_a$ ist ein Maß für die Überlappung;

Fig. 15a)  Beispielhafter Signalverlauf eines Eingangssignals, und des tiefpassgefilterten Gleichsignals (DC-Anteil);

Fig. 15b)  Beispielhafter Signalverlauf des hochpassgefilterten Signals (AC-Anteil);

Fig. 16  Modell des adaptiven Filters mit den Eingangsgrößen links und Ausgangsgrößen rechts, das Referenzsignal ist durch $W^A_c$ gekennzeichnet;

Fig. 17    Beispielhafter Verlauf eines Kaiser-BesselFensters mit einer Blocklänge von 256 Punkten;

Fig. 18    beispielhafter spektraler Verlauf der normierten Nutzsignale für die beiden Hellsendekanäle Rot und Infrarot;

Fig. 19    beispielhafte Darstellung der beiden Spektren für Rot und Infrarot-Sendekanäle, wobei Spektralwerte gleicher Frequenzen gegeneinander aufgetragen sind;

Fig. 20a)    schematische Darstellung des Least Squares Fit-Verfahrens zur Minimierung einer vertikalen Distanz zu einer Geraden;

Fig. 20b)    schematische Darstellung des Total Least Squares Fit-Verfahrens zur Minimierung der tatsächlichen Abstände zu einer Geraden;

Fig. 21a)    beispielhafter Verlauf des Quotienten zwischen dem Rot-Sendekanal und dem InfrarotSendekanal zu vier unterschiedlichen Zeitpunkten k2;

Fig. 21b)    beispielhafter Verlauf eines mit der Methode des Complex Total Least Squares Fit-Verfahrens ermitteltes Referenzspektrums;

Fig. 22    beispielhaftes Spektrum eines Signalverlaufs, bei dem die Amplituden der Störung größer sind als die Amplituden der Pulswelle;

Fig. 23    zeigt ein Referenzspektrum (leere Kreise) und die nach Anwendung der spektralen Maske ausgewählten Koeffizienten (volle Punkte);

Fig. 24    beispielhafte Kennlinie einer Kalibrierungsfunktion;

Fig. 25    prinzipielles Blockschaltbild der Hardware eines Pulsoximeters gemäß dem Stand der Technik; und

Fig. 26    schematisierte Darstellung eines Zeitmultiplexverfahrens (TDMA).

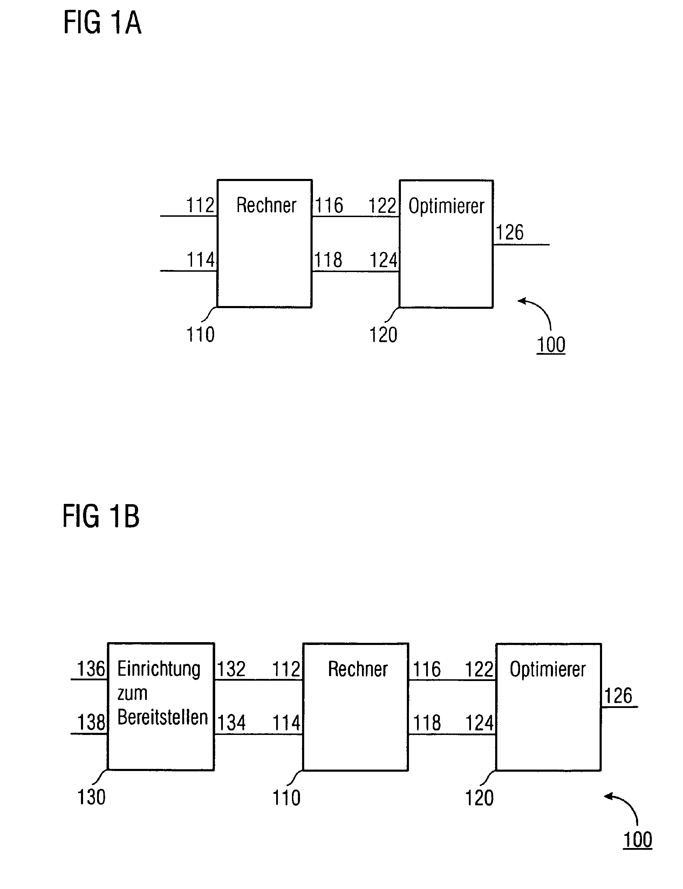

Fig. 1a zeigt eine schematisierte Darstellung eines erfindungsgemäßen Ausführungsbeispiels, mit einer Vorrichtung 100 zum Bestimmen eines Spektralverhältnisses zwischen einem ersten Signal mit einem ersten Spektrum, das von einer biologischen Größe abhängt, und einem zweiten Signal mit einem zweiten Spektrum, das von der biologischen Größe abhängt, wobei das erste Signal und das zweite Signal eine Mehrzahl von Harmonischen eines periodischen Signals haben. Die Vorrichtung zur Bestimmung des Spektralverhältnisses weist einen Rechner 110 auf, der an seinem ersten Eingang 112 das erste Signal empfängt und an seinem Eingang 114 das zweite Signal empfängt, und zur Berechnung eines ersten Wellenverhältnisses, das an einem ersten Ausgang 116 ausgegeben wird, zwischen einem Spektralwert des ersten Spektrums, der eine Frequenz einer Harmonischen der Mehrzahl von Harmonischen hat, und einem Spektralwert des zweiten Spektrums, der die Frequenz der Harmonischen hat, ausgebildet ist. Der Rechner 110 gibt an seinem zweiten Ausgang 118 ein zweites Wellenverhältnis zwischen einem Spektralwert des ersten Spektrums, der eine Frequenz einer anderen Harmonischen der Mehrzahl von Harmonischen hat und einem Spektralwert des zweiten Spektrums, der die Frequenz der anderen Harmonischen hat, aus.

Dem Rechner 110 ausgangsseitig nachgeschaltet ist ein Optimierer 120. Die beiden Ausgänge 116 und 118 des Rechners 110 sind mit zwei Eingängen 122 und 124 des Optimierers 120 verbunden. Der Optimierer 120 ermittelt das Spektralverhältnis unter Verwendung des ersten und zweiten Wellenverhältnisses, wobei der Optimierer ausgebildet ist, um das Spektralverhältnis so zu ermitteln, dass es sich von dem ersten und dem zweiten Wellenverhältnis unterscheidet und ein Optimierungskriterium erfüllt. Das Spektralverhältnis wird dann am Ausgang 126 des Optimierers 120 ausgegeben.

Fig. 1b zeigt eine alternative schematische Darstellung des Ausführungsbeispiels. In Fig. 1b sind die gleichen Komponenten dargestellt, wie in Fig. 1a, die Vorrichtung ist um eine Einrichtung 130 zum Bereitstellen der beiden Signale erweitert. Die Einrichtung 130 zum Bereitstellen des ersten Signals mit dem ersten Spektrum und dem zweiten Signal mit dem zweiten Spektrum verfügt über zwei Ausgänge 132 und 134, die mit den beiden Eingängen 112 und 114 des Rechners verbunden sind. Die Einrichtung 130 zum Bereitstellen der beiden Signale, verfügt weiterhin über zwei Eingänge 136 und 138. Die Einrichtung 130 empfängt an ihren Eingängen 136 und 138 zwei Ursignale, aus denen Spektralanteile ausgeblendet werden, so dass die Einrichtung 130 zum Bereitstellen der Signale an ihren Ausgängen 132 und 134 Signalanteile der Ursignale bereitstellt. Der Rechner 110 empfängt an seinen Eingängen 112 bzw. 114 nun die beiden Signale, die sich aus Signalanteilen der Ursignale zusammensetzen. Die Ursignale können beispielsweise zwei bei der Plethysmographie erfasste optische Empfangssignale sein, die auf Licht unterschiedlicher Wellenlängen (beispielsweise Rot-und Infrarotlicht) zurückgehen, und aus denen für die Bestimmung beispielsweise eines Blutsauerstoffgehalts unrelevante Spektralanteile ausgeblendet werden.

In einem erfindungsgemäßen Ausführungsbeispiel wird eine Lichtquelle, deren Licht in ein Körperteil eines Probanden eingekoppelt wird, und ein Signal von einem Photodetektor empfangen wird, so angesteuert, dass sie in unregelmäßigen Abständen innerhalb einer sich wiederholenden Sequenz den Einzustand einnimmt. Die Unregelmäßigkeit bewirkt dabei, dass im spektralen Bereich des Signals eine Aufweitung stattfindet. Durch die zusätzlichen spektralen Komponenten des Lichtsignals entsteht eine zusätzliche Störsicherheit. Im einfachsten Fall, entstehen zwei Spektrallinien gleicher Höhe. Da die Wahrscheinlichkeit dafür, dass beide Spektralanteile gleichzeitig gestört werden, geringer ist als die Wahrscheinlichkeit, dass ein einzelner Spektralanteil gestört wird, entsteht ein Diversitätsgewinn im Frequenzbereich. Dieser Diversitätsgewinn kann durch eine entsprechende Signalverarbeitung realisiert werden, so dass durch das unregelmäßige Ansteuern der Lichtquellen, eine höhere Störsicherheit und damit eine größere Zuverlässigkeit der Messung eines Vitalparameters erreicht wird. Weiterhin entsteht ein sogenannter Spreizgewinn. Durch das unregelmäßige Ansteuern wird die Energie des Nutzsignals auf mehrere Frequenzanteile gleichmäßig verteilt. Da die Unregelmäßigkeit bekannt ist, können diese Energieanteile im Empfänger wieder kohärent überlagert werden. Störanteile, die bei den gleichen Frequenzen liegen, werden im Empfänger ebenfalls überlagert, da diese allerdings von einander unabhängig sind, geschieht hier eine inkohärente Überlagerung, so dass für das Nutzsignal ein Gewinn entsteht. Ein schmalbandiger Störer, der sich dem Nutzsignal nur bei einem Frequenzanteil überlagert, erfährt im Empfänger eine spektrale Aufweitung analog der des Nutzsignals im Sender, da in beiden Fällen Signalanteile zu unregelmäßigen Zeitpunkten kombiniert werden.

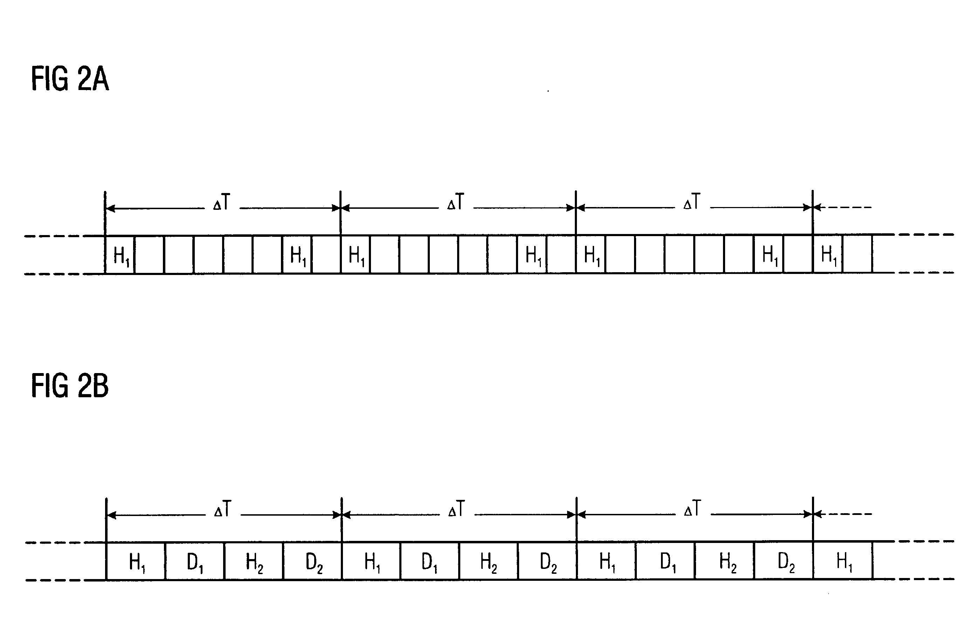

Diese Unregelmäßigkeit der Hellzeit-Dauern ist schematisiert in Fig. 2a dargestellt. Fig. 2a zeigt eine sich wiederholende Sequenz der Dauer $\Delta T$. Innerhalb einer Sequenz nimmt eine Lichtquelle $H_1$ zweimal einen Einzustand ein. Dies ist in Fig. 2a durch die Einträge $H_1$ angedeutet. Während der anderen Zeitpunkte, zu denen im Zeitraster in Fig. 2a keine Einträge vorhanden sind, ist die Lichtquelle ausgeschaltet. Zum Vergleich ist in der Fig. 2b eine Sequenz eines herkömmlichen Pulsoximeters dargestellt. Fig. 2b zeigt ein Zeitmultiplexverfahren (TDMA), bei dem zwei Lichtquellen angesteuert werden. Während einer Sequenz nimmt jede Lichtquelle für einen Zeitschlitz den Einzustand ein. Dies ist in Fig. 2b durch $H_1$ und $H_2$ angedeutet. Während der anderen Zeitdauern, die in Fig. 2b mit $D_1$ und $D_2$ dargestellt sind (D steht für engl. "DARK"), soll keine der beiden Lichtquellen einen Einzustand angenommen haben.

Das unregelmäßige Ansteuern an der Lichtquelle entspricht einer Spreizspektrummodulation. Durch die Spreizspektrummodulation in Kombination mit einer nachgeschalteten adaptiven Filterung werden Signalanteile vermindert, die auf Umgebungslichteinflüsse bzw. auf elektromagnetische Störquellen (z.B. Hochfrequenzchirurgie) zurückzuführen sind und solche die auf die Unterabtastung zurückzuführen sind. Eine nachfolgende Signalverarbeitung erlaubt zudem eine besonders effiziente Messung der Blutsauerstoffsättigung und der Herzfrequenz eines Patienten, wobei mit dem vorliegenden Verfahren auch bei niedriger arterieller Blutvolumenpulsation und bei Bewegung des Patienten zuverlässig gemessen werden kann.

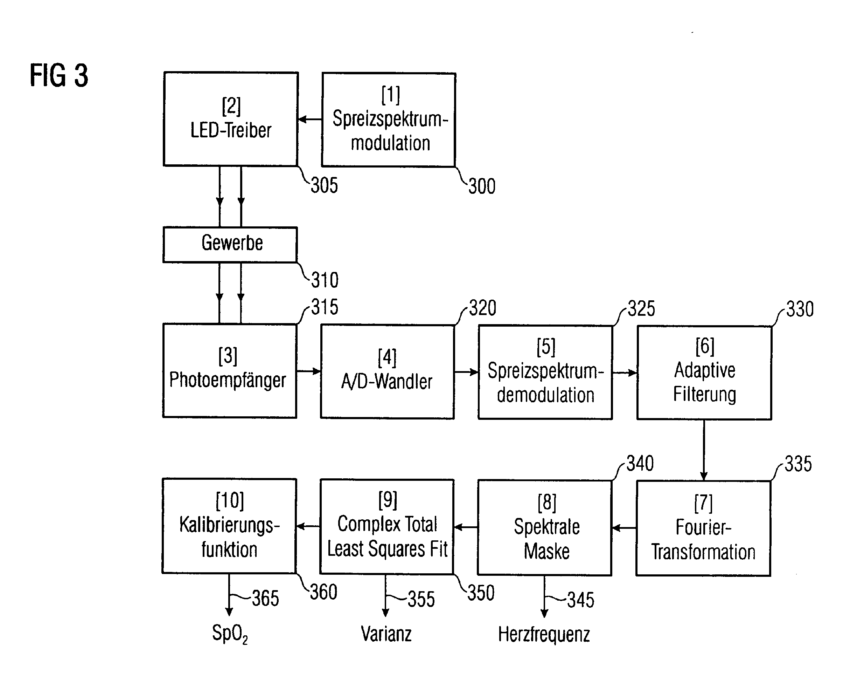

Fig. 3 zeigt eine Realisierung des Ausführungsbeispiels. In Fig. 3 wird zunächst eine Spreizspektrummodulation 300 durch eine LED-Treiberstufe 305 in ein optisches Signal gewandelt. Die LED-Treibereinrichtung 305 koppelt gemäß der empfangenen Spreizspektrummodulation Lichtsignale in ein Gewebe 310 (z.B. in einen Finger) ein, woraufhin die Lichtsignale auf ihren Weg durch das Gewebe moduliert werden und anschließend von einem Photoempfänger 315 empfangen werden. Der Photoempfänger 315 wandelt die empfangenen optischen Signale in elektrische Signale um, und führt diese einer Analog-Digitalwandeleinrichtung 320 zu, die das analoge Signal in ein Digitalsignal umsetzt. Der Analog-Digitalwandeleinrichtung 320 nachgeschaltet ist ein Spreizspektrumdemodulator 325.

Nach der Spreizspektrumdemodulation 325 wird das Signal adaptiv gefiltert 330 und anschließend Fourier transformiert 335. In einem nächsten erfindungsgemäßen Schritt 338, wird ein Referenzspektrum bestimmt, indem erfindungsgemäß eine spektrale Maske 340 auf das Referenzspektrum angewendet wird, woraufhin die Herzfrequenz des Probanden festgestellt werden kann und dann am Ausgang 345 ausgegeben wird. In einem nächsten erfindungsgemäßen Analyseschritt, dem sog. "Complex Total Least Squares Fit"-Verfahren 350 kann nun über eine statistische Analyse im Frequenzbereich eine Varianz der Differenz der unterschiedlichen Spektren, die für Licht unterschiedlicher Wellenlängen bemessen wurden, bestimmt werden und als Zuverlässigkeitsmaß am Ausgang 355 ausgegeben werden. Mit dem Ausgangswert, den die erfindungsgemäße "Complex Total Least Squares Fit"-Einrichtung 350 liefert, kann nun über eine Kalibrierungsfunktion 360 ein zugehöriger Blutsauerstoffgehalt ($SpO_2$-Wert) am Ausgang 365 ausgegeben werden.

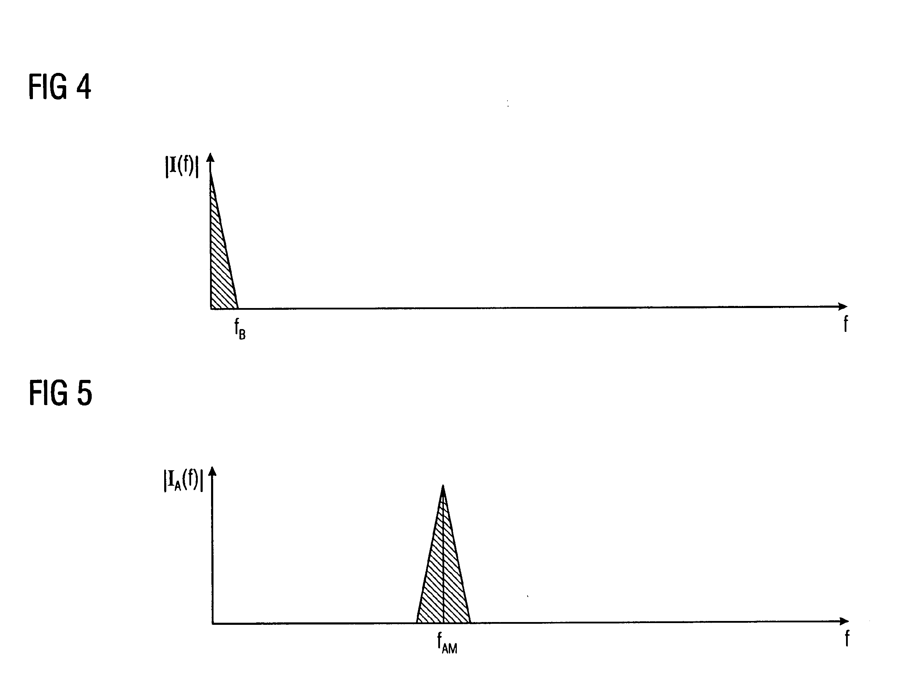

Um die Lichtabsorption des Gewebes 310 mit mehreren Lichtquellen 305 unterschiedlicher Wellenlängen und mittels eines breitbandigen Photoempfängers 315 messen zu können, benötigt man ein Modulationsverfahren, bestehend aus dem Modulator 300 und dem Demodulator 325. Um Störungen besser zu unterdrücken, wird das Spreizspektrumverfahren verwendet. Diesem Modulationsverfahren liegt zugrunde, dass durch die Unregelmäßigkeit der Hellzeit-Dauern das Spektrum des Basisbandsignals gespreizt oder aufgeweitet wird. Dieser Effekt wird durch die Figuren 4 bis 9 verdeutlicht.

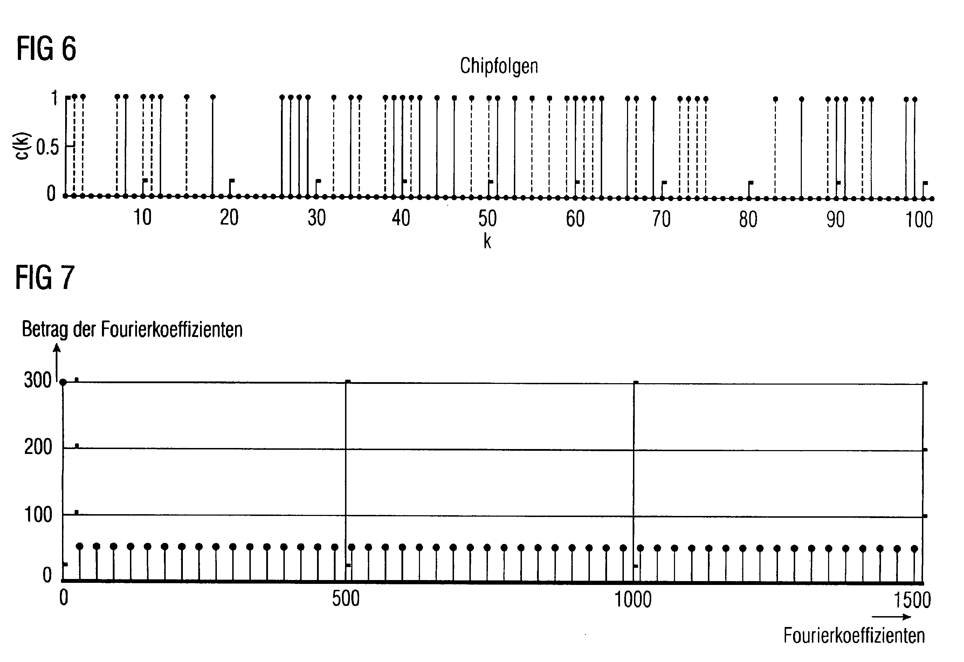

Fig. 4 zeigt zunächst ein Spektrum $|I(f)|$ eines Basisbandsignals, dessen Grenzfrequenz als $f_B$ bezeichnet ist. Bei herkömmlichen Modulationsverfahren, wie z.B. der Amplitudenmodulation wird das Spektrum des Basisbandsignals in einen Frequenzbereich verschoben, der für die Übertragung besser geeignet ist. Fig. 5 illustriert diesen Fall und zeigt das verschobene Spektrum $|I_A(f)|$. Ein solches Spektrum resultiert, wenn das Basisbandsignal mit einer höheren Trägerfrequenz multipliziert wird. Das Spektrum des Basisbandsignals bleibt dabei von seiner Form und Energie her unverändert. Wird dieses Signal nun von einem Störer überlagert so ist diese Störung durch Demodulation, also durch Zurückverschieben aus dem Übertragungsband in das Basisband nicht zu unterdrücken. Im Falle der Spreizspektrummodulation wird jedem Sendekanal, darunter werden die Sendelichtsignale einer Wellenlänge verstanden, einer zuvor berechneten, sogenannten Chipfolge zugeordnet. Eine Chipfolge besteht aus einer endlichen Sequenz von Einsen und Nullen, die typischerweise in einer um das Hundertfache höheren Frequenz getaktet sind als vergleichsweise bei einem TDMA-Konzept. Die Taktfrequenz liegt etwa bei 3kHz.

Die Chipfolgen müssen aus mathematischer Sicht bestimmte Eigenschaften erfüllen, um die gewünschte Spreizwirkung des Störsignals zu erzielen und die Rekonstruktion der Plethysmogramme, sowie der Umgebungslichtkanäle zu ermöglichen. Grundsätzlich müssen die Chipfolgen orthogonal sein, um bei der Demodulation eine Kanaltrennung realisieren zu können und damit eine Demodulation ohne Übersprechen ermöglicht wird.

Fig. 6 zeigt eine schematische Darstellung zweier orthogonaler Chipfolgen, wobei die Länge einer Chipfolge im hier betrachteten Ausführungsbeispiel gleich 101 Chips ist. In Fig. 6 ist ein Zeitstrahl einer Dauer von 101 Chipdauern dargestellt. Über diese 101 Chipdauern sind die Werte zweier Chipfolgen $c(k)$ aufgetragen. Im Diagramm sind die beiden Chipfolgen durch gestrichelte bzw. durchgezogene Linien unterschieden. Immer wenn eine Chipfolge den Wert 1 annimmt, bedeutet dies, dass die zugehörige Lichtquelle in den Einzustand gebracht wird. In Fig. 6 lässt sich sehr deutlich erkennen, dass die beiden Chipfolgen orthogonal sind, d.h. dass die beiden zugeordneten Lichtquellen niemals gleichzeitig den Einzustand einnehmen. Prinzipiell ist es auch möglich, Chipfolgen einzusetzen, die gleichzeitig eine 1 bewirken, bzw. der Einsatz anderer Folgen mit anderen Eigenschaften ist möglich. Hier ist die Eigenschaft der Folgen hervorzuheben, dass die einzelnen Hellzeitdauern in unregelmäßigen Abständen auftreten, so dass eine spektrale Spreizung erreicht wird. Weiterhin ist in Fig. 6 deutlich zu erkennen, dass die einzelnen HellZeitdauern innerhalb einer Sequenz unregelmäßig angeordnet sind, und dass es Zeitpunkte gibt, zu denen beide Chipfolgen der Wert 0 annehmen, d.h. in der Realisierung beide Lichtquellen ausgeschaltet sind.

Eine weitere wichtige Eigenschaft der Chipfolgen ist, dass ihr Spektrum möglichst gleichverteilt sein sollte, damit sich die Signalenergie möglichst gleichmäßig auf einen möglichst breiten Frequenzbereich verteilt.

Fig. 7 zeigt das Spektrum, d.h. den Frequenzbereich einer der in Fig. 6 dargestellten Chipfolgen. In Fig. 7 ist deutlich zu erkennen, dass das Spektrum einer solchen Folge gleichverteilt ist, d.h. das Spektrum setzt sich aus äquidistanten gleichen Werten zusammen. Der hohe Gleichanteil, der sich durch den überhöhten Wert bei der Frequenz 0 darstellt, lässt sich dadurch erklären, dass die Chipfolge nur die Werte 0 und 1 annehmen kann. Dadurch ist die Folge nicht mittelwertfrei. Das Spektrum einer Chipfolge kann also wie ein "Kamm" aus äquidistanten Trägern gleicher Amplitude betrachtet werden. Die spektrale Gleichverteilung einer Chipfolge hat zur Konsequenz, dass ein schmalbandiger Störer nach der Demodulation in ein breitbandiges Rauschen gespreizt wird. Die beiden LEDs werden in der Realisierung des Ausführungsbeispiels, wie es in Fig. 3 dargestellt ist, mit dem in Fig. 6 dargestellten Chipsequenzen angesteuert.

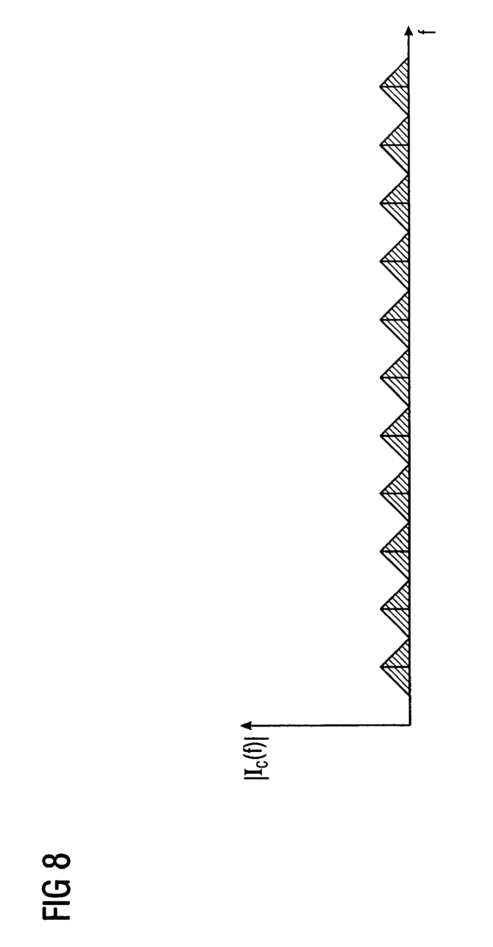

Fig. 8 zeigt die schematische Darstellung des Signals aus Fig. 4 im Übertragungsband $|I_c(f)|$. Das Basisbandsignal, wie es in Fig. 4 dargestellt ist, behält seine spektrale Form bei, seine Energie wird aber auf viele Frequenzen verteilt. Dieser Vorgang wird auch als Spreizen bezeichnet. Wird das in Fig. 8 dargestellte Signal nun durch einen schmalbandigen Störer gestört, so erfährt dieser eine Spreizung bei der Demodulation, wohingegen die Energieanteile des Signals aus Fig. 8 sich im Basisband wieder kohärent überlagern. Die Demodulation entspricht dabei einer erneuten Multiplikation mit der entsprechenden Chipfolge. Das Ergebnis der Multiplikation wird dann über eine Chipfolgenlänge hinweg aufsummiert. Multipliziert man also ein Empfangssignal mit einer der Chipfolgen, wie sie in Fig. 6 dargestellt sind, so lässt sich aus Fig. 6 leicht erkennen, dass aus dem Empfangssignal durch die Multiplikation nur diejenigen Empfangssignalwerte ausgeblendet werden, die zu einem Zeitpunkt empfangen werden, die einer Eins in der jeweiligen Chipfolge entsprechen. Diese einzelnen Signalanteile werden dann über eine Chipfolge hinweg aufsummiert, wodurch sie sich kohärent, d.h. konstruktiv, überlagern. Ein sich dem Empfangssignal überlagertes Störsignal wird ebenfalls nur zu den entsprechenden Zeitpunkten eingeblendet. Auch die Störsignale werden zu den jeweiligen Zeitpunkten abgetastet und über die Länge einer Chipfolge hinweg aufsummiert. Die Störsignale überlagern sich jedoch zu den Abtastzeitpunkten nicht kohärent, so dass diese über die Entspreizung hinweg tatsächlich eine Spreizung erfahren, nämlich die Multiplikation mit der Chipfolge, so dass nach der Demodulation diese Signale nur noch gedämpft vorliegen.

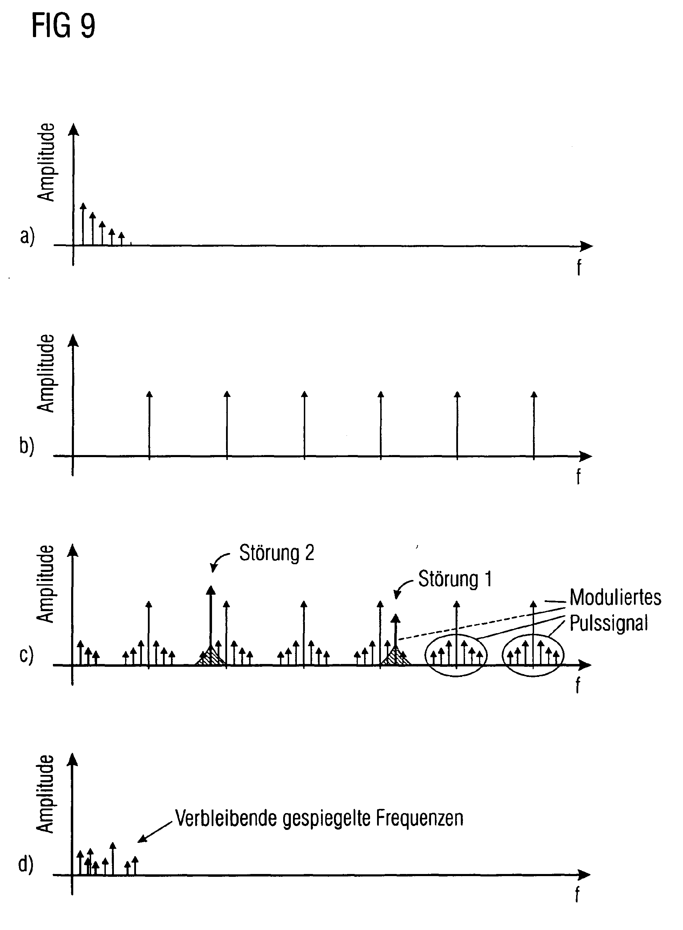

In den Fig. 9a)-d) ist die Operation des Spreizens noch einmal im Frequenzbereich dargestellt. Fig. 9a) zeigt das Spektrum eines Signals im Basisband. Fig. 9b) zeigt das Spektrum einer Chipfolge, das idealerweise spektral gleichverteilt ist. In der Fig. 9c) ist das gespreizte Basisbandsignal zu sehen, welches nun Energieanteile bei jeder einzelnen Frequenz der Chipfolge aufweist. Die Energie des Basisbandsignals wurde aufgespreizt auf die Frequenzen die in der Chipfolge enthalten sind. In der erfindungsgemäßen Realisierung wird das Signal in dieser Form aus dem Gewebe durch den Photosensor empfangen, das eigentliche Nutzsignal, wurde dann durch das Gewebe auf das gespreizte Signal aufmoduliert. Die Fig. 9c) zeigt ferner zwei Störungen, "Störung 1" und "Störung 2". Es handelt sich bei den beiden Störungen um schmalbandige Störer, wie sie z.B. von Leuchtstoffröhren oder Hochfrequenzskalpellen verursacht werden können. Fig. 9d) zeigt das Spektrum des Signals nach der Demodulation bzw. nach dem Entspreizen. Es ist zu erkennen, dass das Basisbandsignal rekonstruiert wurde und das zusätzliche Frequenzen der Störsignale im Basisband hinzukamen. Fig. 9d) zeigt weiterhin, dass die verbleibenden Frequenzen der Störung deutlich geringere Amplituden aufweisen als die ursprüngliche Störung selbst, was auf die Spreizung des Störsignals zurückzuführen ist.

Legendre-Folgen sind Chipfolgen, die die hier geforderten Eigenschaften erfüllen und gute Auto- und Kreuzkorrelationseigenschaften besitzen. Die Folgen modulieren zwei Hell- und zwei Dunkel-Sendekanäle in der betrachteten Realisierung des Ausführungsbeispiels. Die spektralen Eigenschaften aller Folgen sind identisch und erfüllen die erforderte Gleichverteilung im spektralen Bereich. Ferner werden insgesamt vier Folgen betrachtet, wobei die vier Folgen untereinander orthogonal sind, das bedeutet, keine zwei Folgen nehmen gleichzeitig den Wert 1 an. Prinzipiell ist auch der Einsatz anderer Folgen denkbar, die Eigenschaft der Unregelmäßigkeit der Hellzeitdauern ist hervorzuheben, dies setzt nicht voraus, dass zu einem Zeitpunkt jeweils nur eine Folge eine Hellzeitdauer haben kann. Zwei der vier Folgen werden in einer Realisierung des Ausführungsbeispiels verwendet, um zwei LEDs verschiedener Wellenlänge (Rot und Infrarot) anzusteuern, die beiden verbleibenden Folgen dienen dazu, Umgebungslichtkanäle zu erfassen, d.h. sie entsprechen Dunkelkanälen.

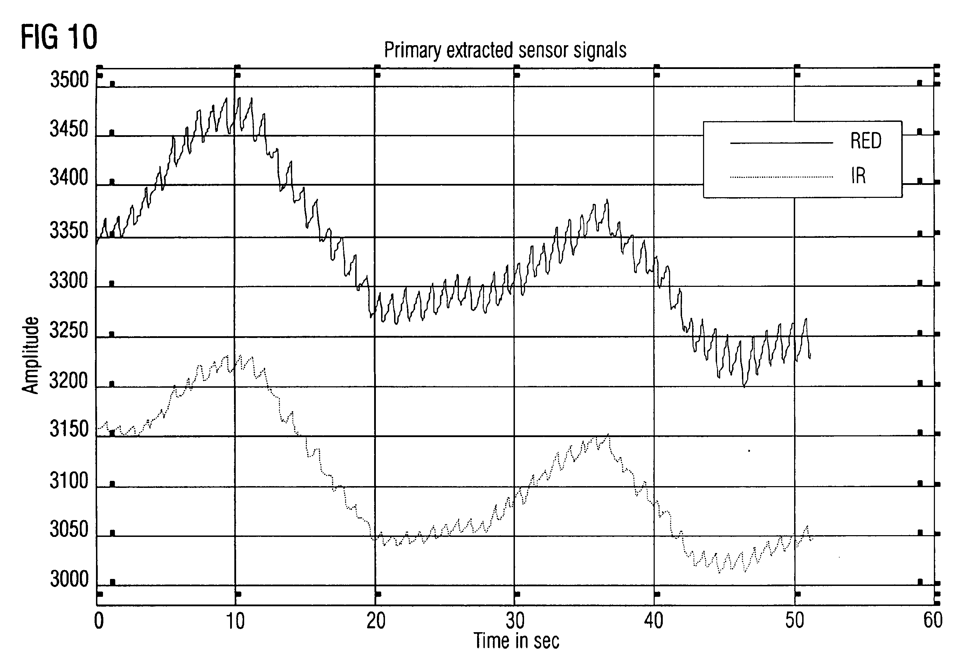

Über den LED-Treiber 305 aus Fig. 3 werden nun die LEDs als monochromatische Lichtquellen angesteuert. Das mit den Chipfolgen modulierte Licht der LEDs tritt durch eine Gewebeschicht und erfährt dabei abhängig von der Wellenlänge der Lichtquelle eine entsprechende Dämpfung. Am Photoempfänger 315 trifft die durch das Gewebe gedämpfte Strahlung der LEDs auf, wird dort zu einem proportionalen Photostrom gewandelt und anschließend mit einem Analog-Digitalwandler 320 synchron zum Takt des Modulators 300 abgetastet. Die Synchronität zwischen Modulator im Sender und AD-Wandler bzw. Demodulator im Empfänger kann optional durch eine Kontrolleinrichtung, die über Steueranschlüsse sowohl Sender als auch Empfänger einen Takt vorgibt, gelöst werden. Das synchronabgetastete Signal wird dem Spreizspektrumdemodulator 325 zugeführt. Der Spreizspektrumdemodulator 325 trennt mit der Demodulation das Signal des Photoempfängers in einzelne Kanäle auf. In einer praxisnahen Implementierung sind dies zwei Pulskanäle für Rot- und Infrarot-LEDs, sowie zwei Kanäle für die Messung des Umgebungslichts. Fig. 10 zeigt zwei beispielhafte Signalverläufe, wobei der untere der roten LED und der obere der infraroten LED entspricht. In Fig. 10 ist zu erkennen, dass beide Signale von einem höherfrequenten Signalanteil überlagert sind, der vom Pulssignal des Probanden stammt, dass beide Signale einen hohen Gleichanteil aufweisen und dass beide Signale einen niederfrequenten Störanteil haben, der beispielsweise durch Umgebungslichtveränderung aufgrund von Bewegungen des Probanden entstanden sein könnte.

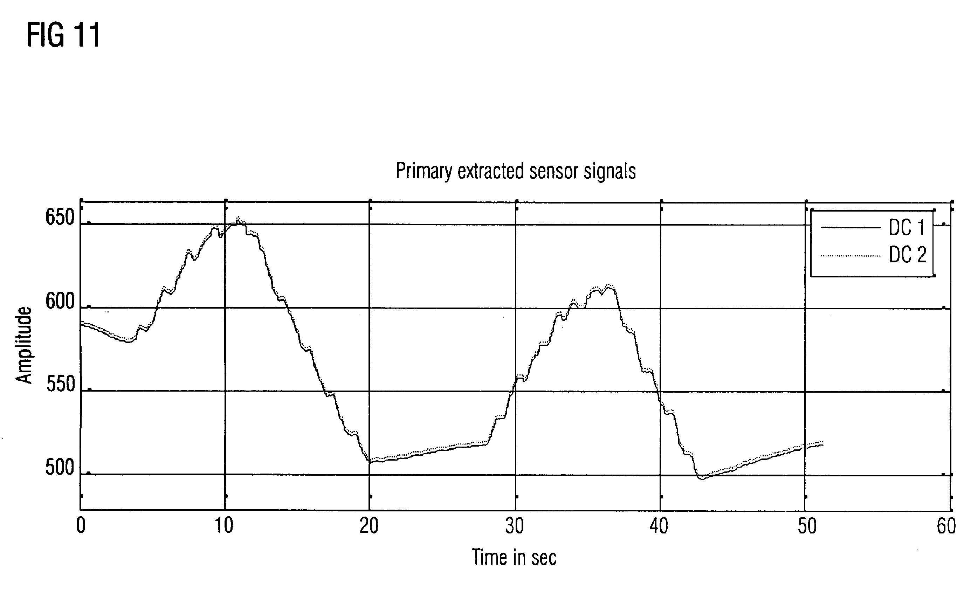

Fig. 11 zeigt zwei beispielhafte Signalverläufe für die beiden Dunkelkanäle. Auch in diesen beiden Signalen ist der hochfrequente Anteil zu erkennen, der vom Pulssignal des Probanden stammt, sowie ein Störanteil, der auf Umgebungslichtveränderungen zurückzuführen ist. Der Gleichanteil in Fig. 11 ist entsprechend geringer als der Gleichanteil in Fig. 10, da die beiden Lichtquellen während der Dunkelkanalphasen abgeschaltet sind. Um nun die Einflüsse des Umgebungslichts aus den Hell-Sendekanälen herauszurechnen, wird der Mittelwert der beiden Umgebungslichtkanäle von den beiden Hell-Sendekanälen subtrahiert, um den niederfrequenten, unterhalb der beiden Abtastfrequenzen liegenden Anteil an Umgebungslicht aus dem gemessenen Signal zu entfernen. Das Bilden des Mittelwertes entspricht dem erfindungsgemäßen Bilden einer gewichteten Summe, wie es erfindungsgemäß durch eine Einrichtung 150 zum Bilden einer gewichteten Summe in Fig. 1b) realisiert wird.

Zur Demodulation wird für jede Chipfolge ein sog. Matched Filter (englisch: angepasstes Filter) zur Extraktion der Sendekanäle aus dem Empfangssignal verwendet. Ein solches Matched Filter ist eine Realisierung des Spreizspektrummodulators 325 aus Fig. 3 und lässt sich als mathematische Operation mit einer Chipfolge beschreiben. Das Sensorsignal wird dabei zyklisch mit der Chipfolge multipliziert und das Ergebnis über jeweils eine Chipfolgenlänge aufsummiert. In der hier beschriebenen Realisierung des Ausführungsbeispiels sind dies die jeweiligen Legendre-Folgen. Das Matched Filter realisiert mathematisch gesehen ein Skalarprodukt, zwischen der Chipfolge und dem Empfangsvektor, d. h. dem gesampelten Empfangssignal. Sender und Empfänger sind dabei synchronisiert. Das Skalarprodukt führt zu einer blockweisen Entspreizung eines Sendekanals ins Basisband. Zugleich entsteht eine Unterabtastung mit einem Faktor, der der Länge der Chipfolge entspricht für das Nutzsignal. Um Aliasing zu vermeiden, muss die Bandbreite des Signals vor jeder Unterabtastung reduziert werden. Demnach wird ein Anti-Aliasingfilter benötigt, welches zusammen mit dem Matched Filter zu einem Filter integriert werden kann.

Untersuchungen haben gezeigt, dass Störungen starker Amplitude überwiegend auf künstliche Beleuchtung zurückzuführen sind. In Europa beträgt die Netzfrequenz 50Hz, demnach liegt die Grundwelle der Leistung (bzw. der Intensität) bei 100Hz, und deren Oberwellen liegen entsprechend bei den Vielfachen von 100Hz. Je nach Intensität der Störung, reicht die Dämpfung des Extraktionsfilters im Sperrbereich nicht aus. Aufgrund dieser Erkenntnis können die Frequenzen, die einem Vielfachen von 100 Hz entsprechen, durch Anpassung der Eigenschaften des Extraktionsfilters (kombiniertes Filter) unterdrückt werden.

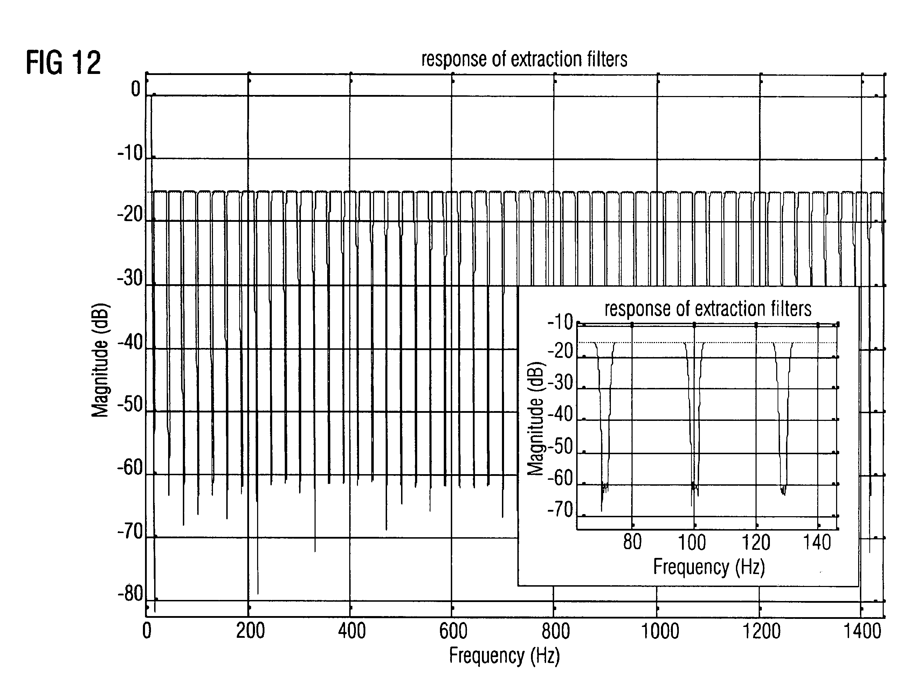

Fig. 12 zeigt beispielhaft eine Übertragungsfunktion eines Extraktionsfilters mit 15dB Dämpfung, bei dem zusätzlich die Störer bei Vielfachen von 100Hz unterdrückt werden. Das Extraktionsfilter beinhaltet also bereits ein für die Unterabtastung notwendiges Tiefpassfilter, und zugleich ein Matched Filter zum Entspreizen des Spreizsignals aus dem Übertragungsband in das Basisband. Ein Filter, das eine Unterabtastung realisiert, wird auch Sub-Sampler genannt, das Matched Filter zum Entspreizen des Spreizsignals wird auch Korrelator genannt, da es eine vorgegebene Chipfolge mit dem Empfangssignal korreliert.

Nach der Extraktion aus dem Empfangssignal, liegen die extrahierten und unterabgetasteten Signale vor. Der Grad der Unterabtastung richtet sich dabei nach der Chipfolgenlänge. Pro Chipfolgenlänge entsteht durch das Matched Filter ein Abtastwert (Sample) des Nutzsignals. Durch die Verwendung mehrerer orthogonaler Chipfolgen entstehen mehrere Kanäle während einer Chipfolgendauer, im erfindungsgemäßen Ausführungsbeispiel gibt es vier Kanäle, zwei Hell-Sendekanäle der Rot und Infrarot-LED, sowie zwei Dunkel-Sendekanäle, während deren keine der Sendelichtquellen einen Einzustand annimmt, und die zur Umgebungslicht- und Störungskompensation verwendet werden.

Weiterhin werden durch das Extraktionsfilter die Störungen oberhalb des Nutzbandes, also Störungen oberhalb der halben Abtastfrequenz mit 15dB ins Nutzband gespiegelt. Die Dämpfung der Störungen oberhalb der halben Abtastfrequenz hängt von der Chipfolgenlänge ab. In der erfindungsgemäßen Realisierung des Ausführungsbeispiel wurde eine Chipfolgenlänge von 101 Chips gewählt, was zu 15dB Dämpfung für Störungen oberhalb der halben Abtastfrequenz führt. Gleichzeitig realisiert das Filter eine zusätzliche Dämpfung aller Frequenzen, die ein Vielfaches von 100 Hz aufweisen. Fig. 12 zeigt eine beispielhafte Übertragungsfunktion eines Extraktionsfilters.

Nach dem Extraktionsfilter liegen die Nutzsignale im Basisband vor. Um die Einflüsse des Umgebungslichtes zu vermindern, erfolgt erfindungsgemäß eine Subtraktion des Umgebungslichtanteils vom Nutzsignal, gemäß der ersten Einrichtung 110 zum Bereitstellen des zeitdiskreten Signals in Fig. 1b). Zusätzlich erfolgt eine Generierung eines Differenzsignals für den adaptiven Filter 330, gemäß der zweiten Einrichtung 120 zum Bereitstellen des ersten und des zweiten zeitdiskreten Referenzsignals und der Subtrahiereinrichtung 130, wie in den Fig. 1a) und 1b) dargestellt. Zur Umgebungslichtsubtraktion wird zunächst aus den Dunkelkanälen ein Mittelwert gebildet, der dann von den Hellsendekanälen subtrahiert wird. Je nachdem welche Art von Chipfolgen verwendet werden, bzw. wie die Spektren der einzelnen Chipfolgen ausgebildet sind, kann es vorteilhaft sein, nicht den exakten Mittelwert der Dunkelkanäle zu bestimmen, sondern die Dunkelkanäle linear zu gewichten. In der Realisierung des erfindungsgemäßen Ausführungsbeispiels werden Legendre-Folgen der Länge 101 Chips verwendet. Bei dieser Realisierung ergibt sich eine optimale Gewichtung der Dunkelkanäle von 47,5% zu 52,5%.

Zur weiteren erfindungsgemäßen Signalverarbeitung ist es wichtig, zwischen zwei Frequenzbändern zu unterscheiden, in die ein Störer fallen kann. Zum einen existiert das Band unterhalb der halben Abtastfrequenz, das Nutzband. Zum anderen existiert das Band oberhalb dieser Frequenz, das Übertragungsband. Störungsbedingte Frequenzkomponenten, die in das Nutzband fallen, können mittels Dunkelphasensubtraktion aus den beiden Nutzsignalen (Hellsendekanäle der Rot und Infrarot-LEDs) entfernt werden. Die Signale dieser Frequenzen sind sowohl in Phase als auch in Amplitude gleich, und treten deshalb nicht in der Differenz der beiden Dunkelkanäle, dem Differenzsignal, auf. Ein Störer im Nutzband (oder Basisband) ergibt demnach durchgehend 0 für das Differenzsignal. Ein Störer im Nutzband könnte eine Lichtquelle sein, die durch das Gewebe vom Fotosensor erfasst, und deren Intensität mit den Volumenänderungen des arteriellen Blutes moduliert wird. Diese Anteile sollen jedoch nicht aus dem Nutzsignal ausgefiltert werden, da sie die gewünschte Information (den pulsatilen Anteil) enthalten.

Im Gegensatz dazu könnte ein Störer in das Übertragungsband fallen. In diesem Fall setzt die Dämpfung des Extraktionsfilters ein, was zunächst dazu führt dass die Störung gedämpft in das Nutzband fällt. In der Realisierung des Ausführungsbeispiels beträgt diese Dämpfung 15dB. Zusätzlich erfahren Signale dieser Frequenzen eine Phasenverschiebung, die für jeden Kanal unterschiedlich ist. Dieser Effekt ist zurückzuführen auf das sequentielle Abtasten, obwohl die orthogonalen Chipfolgen in einander verschachtelt sind, vgl. Fig. 6, realisieren sie Abtastwerte zu unterschiedlichen Zeitpunkten. Für Signale oberhalb der halben Abtastfrequenz führt dies zu einer Phasenverschiebung der unterabgetasteten Signale in den einzelnen Kanälen.

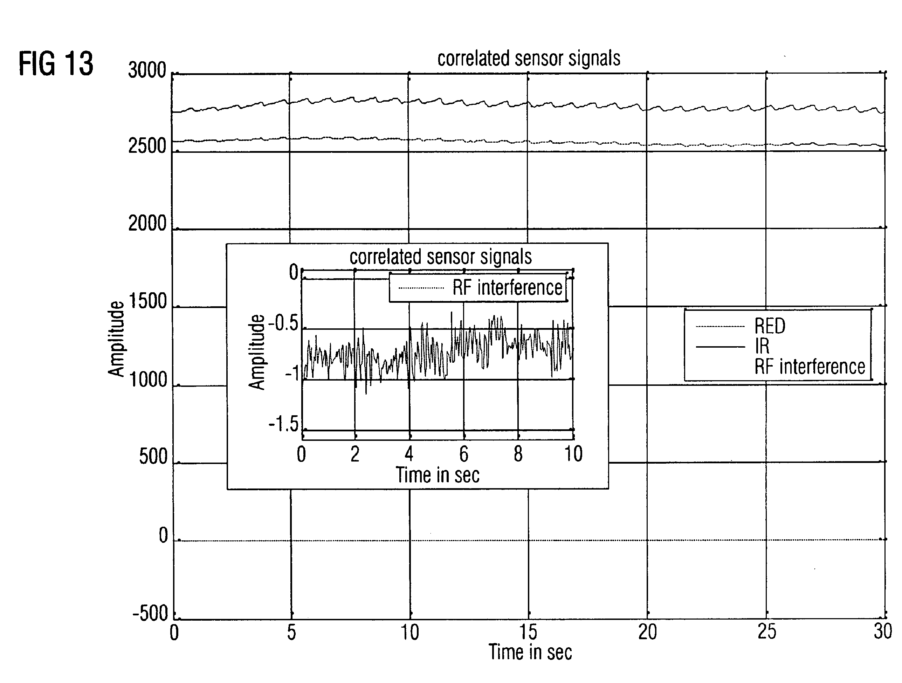

Damit ergibt die Differenz der beiden Dunkelsendekanäle (das Differenzsignal) keine Auslöschung dieser Signale, sondern ein Signal, dessen Frequenzkomponenten die gespiegelten Frequenzen des Störers aus dem übertragungsband enthalten. Dieses Signal dient nun als Differenzsignal für ein erfindungsgemäßes adaptives Filter 330, um auch die verbleibenden Störungen aus dem Übertragungsband zu vermindern. Die Umgebungslichtsubtraktion entfernt also die Störer aus dem Nutzband, enthält jedoch auch phasenverschobene Störanteile aus dem Übertragungsband. Nachdem die Störungen aus dem Übertragungsband durch die Extraktion eine Dämpfung erfahren haben, werden nun Anteile dieser Störung durch die Umgebungslichtsubtraktion dem Nutzsignal wieder zugeführt. Dadurch ergibt sich nicht die volle Dämpfung für die Störungssignale aus dem Übertragungsband, sondern ein geringerer Wert. In der Realisierung des erfindungsgemäßen Ausführungsbeispiels liegt die Dämpfung durch das Extraktionsfilter zunächst bei 15dB, die jedoch durch die Umgebungslichtsubtraktion um 3dB wieder vermindert wird, so dass sich insgesamt für Störer aus dem Übertragungsband eine Dämpfung von 12dB ergibt. Fig. 13 zeigt zwei beispielhafte Signalverläufe für die beiden Sendekanäle, Rot und Infrarot LEDs, von denen das Umgebungslichtsignal subtrahiert wurde. Weiterhin ist in Fig. 13 ein beispielhaftes Differenzsignal vergrößert dargestellt.

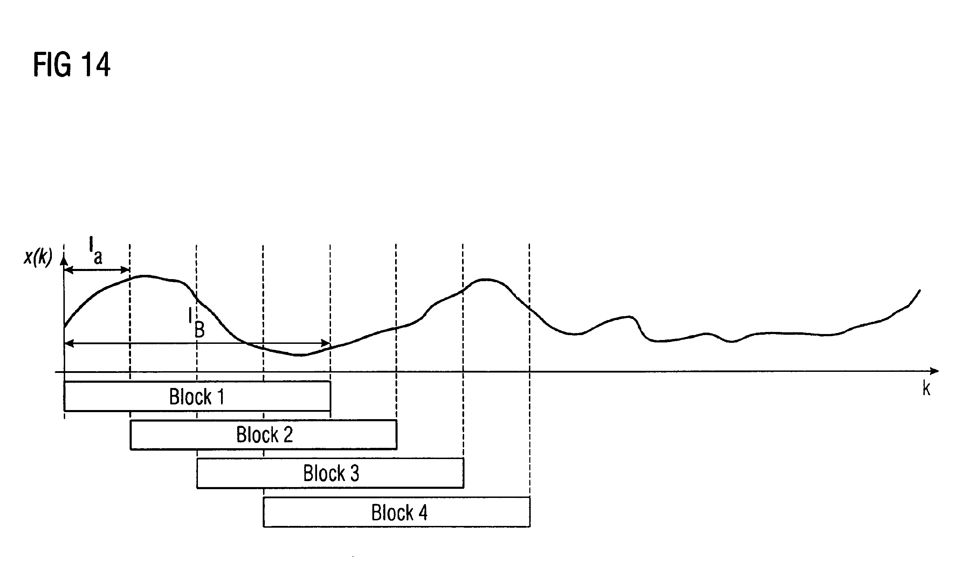

Zur weiteren Signalverarbeitung erfolgt zunächst eine Blockbildung für die einzelnen Signale. Die Signale werden dazu in Blöcke gleicher Länge unterteilt, wobei sich die einzelnen Blöcke überlappen. Fig. 14 verdeutlicht die Blockbildung zur weiteren Signalverarbeitung.

Dabei werden aus den Abtastwerten eines Nutzsignals Blöcke der Länge $I_B$ gebildet, wobei alle $I_a$ Samples ein neuer Block gebildet wird.

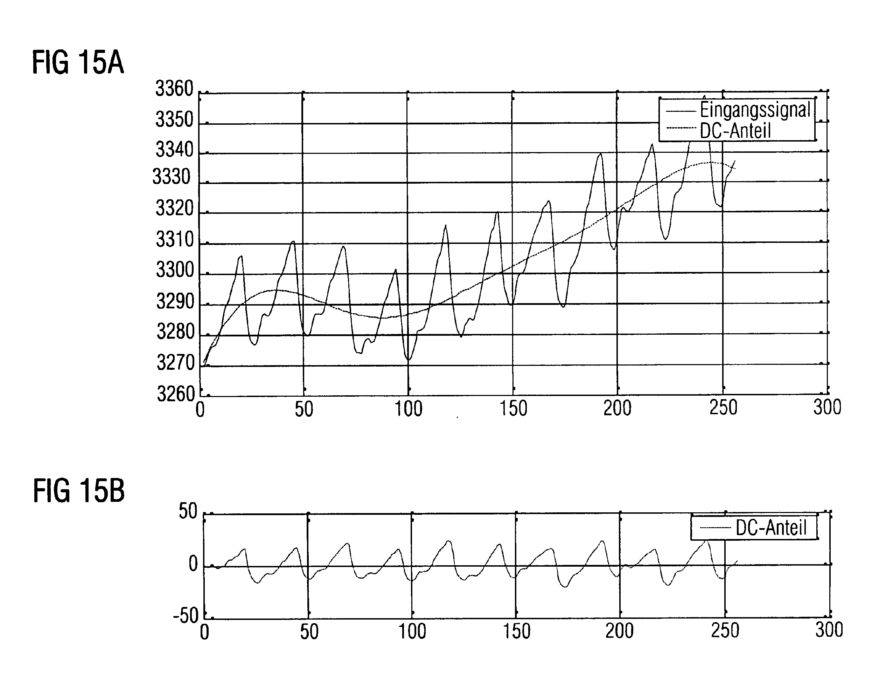

Die Nutzsignale werden im Anschluss einer Frequenzweiche zugeführt. Die Aufgabe der Frequenzweiche ist die Filterung des Gleichanteils und des pulsatilen Anteils aus den Eingangssignalen. In der Realisierung des erfindungsgemäßen Ausführungsbeispiels liegt die Trennfrequenz der Frequenzweiche etwa bei 0,5Hz. Fig. 15a) zeigt den beispielhaften Verlauf eines Eingangssignals, das der Frequenzweiche zugeführt wird. Weiterhin ist in Fig. 15a) der tiefpassgefilterte Anteil (DC-Anteil) des Eingangssignals dargestellt. Fig. 15b) zeigt den dazugehörigen Hochpassanteil (AC-Anteil) des Eingangssignals. Die weitere Signalverarbeitung bezieht sich nur noch auf den Hochpassanteil des Eingangssignals.

Die hochpassgefilterten Nutzsignale werden nun einem adaptiven Filter 330 zugeführt. Die Aufgabe dieses Filters, das auch Interference Canceller genannt wird, ist es Störungen zu vermindern, die im Übertragungsband lagen und nach der Demodulation gedämpft in das Nutzband gespiegelt worden sind, vgl. Fig. 9d). Aus den Dunkel-Sendekanälen wurde das Differenzsignal durch Subtraktion gebildet, das die Frequenzen der Störung im Nutzband enthält. Das Differenzsignal unterscheidet sich in Phase und Amplitude von den den Nutzsignalen überlagerten Störungen. Der Phasenunterschied kommt durch das zeitlich versetzte Abtasten zustande, der Unterschied in der Amplitude entsteht sowohl durch das zeitlich versetzte Abtasten als auch durch die Subtraktion. Aufgabe des adaptiven Filters ist es deswegen, die unerwünschten Spiegelfrequenzen anhand des Differenzsignals aus den Nutzsignalen herauszufiltern. Dazu wird aus dem Differenzsignal ein Störungssignal konstruiert, das der Störung, die dem Nutzsignal überlagert ist, möglichst nahe kommt. Zur Bestimmung der Koeffizienten für das adaptive Filter 330, gibt es mehrere mathematische Verfahren. Ein bekanntes Verfahren wäre die Koeffizienten des adaptiven Filters 330 derart zu wählen, dass die Abweichung zwischen dem Differenzsignal und dem Nutzsignal minimiert wird. Zur Bestimmung der Koeffizienten wäre auch hier das Complex Total Least Squares Fit-Verfahren zu nennen.

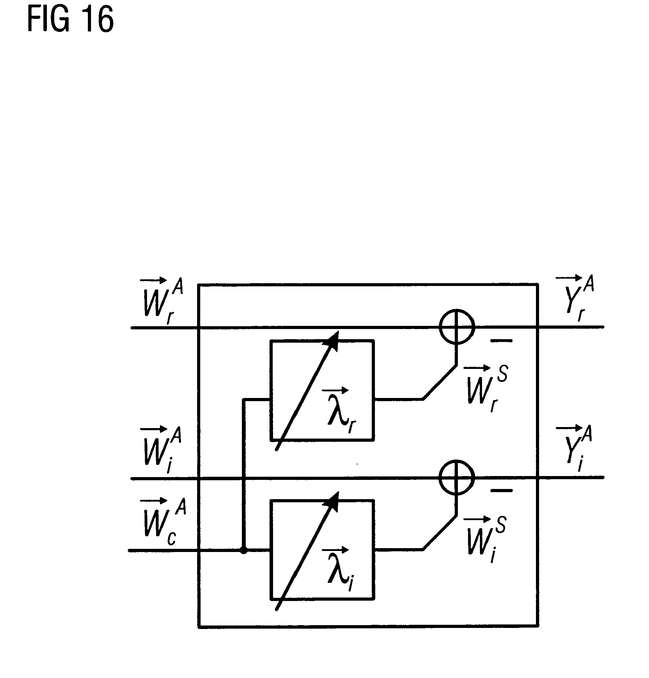

Fig. 16 zeigt das Modell des adaptiven Filters mit den zeitdiskreten Eingangsgrößen $\vec{w}^A_r$ und $\vec{w}^A_i$ für die beiden Eingangssignale der Hellsendekanäle für Rot und Infrarot, wobei A hier anzeigt, dass die Eingangssignale hochpassgefiltert sind. Prinzipiell werden die im folgenden beschriebenen Operationen auf beide Eingangsgrößen getrennt angewendet, da die betrachtete Störung auch in ihnen phasenverschoben vorliegt. Das Differenzsignal ist als Matrix $W^A_c$ ebenfalls hochpassgefiltert, und bildet die Basis zur Bestimmung der adaptiven Filterkoeffizienten $\vec{\lambda}_r$ und $\vec{\lambda}_i$. Die Matrix $W^A_c$ ergibt sich dabei aus Blöcken des Differenzsignals, die hochpassgefiltert sind um den Gleichanteil zu entfernen. Die Spalten der Matrix bilden dabei jeweils einen Block von Abtastwerten, z.B. der Länge 256. Dieser Block wird von Spalte zu Spalte in der Matrix jeweils um ein Sample eingerückt, die Matrix besitzt so viele Spalten, wie es Koeffizienten für den adaptiven Filter gibt. Die Matrix kann beschrieben werden als

$$\left[W^A_c\right]_{ij}(k) = w^A_c(i + k \cdot l_a + j)$$
$$\forall j \in \left\{0,1,...,N_{ifc}\right\}$$
$$\forall i \in \left\{0,1,...,l_B - 1\right\}, \tag{1}$$

wobei $w^A_c$ die hochpassgefilterten Elemente des Differenzsignals darstellen, k eine diskrete Laufvariable der Blockbildung ist, $l_a$ die Sprungkonstante bei der Blockbildung, $l_B$ die Blocklänge und $N_{ifc}$ die Filterordnung, d.h. die um eins verringerte Anzahl der Filterkoeffizienten des adaptiven Filters, bzw. Interference-Cancellers. Der Ausgang des adaptive Filters ist also eine gewichtete Summe aus jeweils um ein Sample verschobenen Blöcken der Differenzsignals. Mit dem adaptiven Filter werden zunächst die Störvektoren rekonstruiert, die in Fig. 16 mit $\vec{w}^S_r$ und $\vec{w}^S_i$ bezeichnet sind und sich den Einganssignalen $\vec{w}^A_r$ und $\vec{w}^A_i$ überlagern. Durch Subtraktion werden dann die Störeinflüsse in den Eingangssignalen $\vec{w}^A_r$ und $\vec{w}^A_i$ vermindert, wie in Fig. 16 dargestellt ist.

Gesucht ist zunächst eine Linearkombination der Basis $W^A_c$, die das Eingangssignal am besten wiedergibt, also eine Rekonstruktion der Störung, so wie sie einem Eingangssignal $\vec{w}^A$ überlagert wurde.

$$\vec{w}^A \overset{!}{=} W_c^A \vec{\lambda} \quad . \qquad (2)$$

Es existieren mehr Gleichungen als Unbekannte, insofern als dass davon ausgegangen wird, dass das adaptive Filter weniger Koeffizienten aufweist als die zu verarbeitende Blocklänge. Deshalb gibt es in diesem Fall keine konkrete Lösung. Ein Vektor , der am besten in das überbestimmte Gleichungssystem passt ist gesucht:

$$\left\| W_c^A \vec{\lambda} - \vec{w}^A \right\| \longrightarrow Minimum \; . \qquad (3)$$

Dieses Problem lässt mit Hilfe der Pseudoinversen angehen. Damit erhält man mit dem Vektor eine Linearkombination von $W_c^A$, mit der sich die Störung beschreiben lässt:

$$\left( W_c^A \right)^{\#} \vec{w}^A = \vec{\lambda} \; . \qquad (4)$$

Damit lässt sich der Störer aus dem Eingangssignal rekontruieren:

$$\vec{w}^s = W_c^A \left( W_c^A \right)^{\#} \vec{w}^A \; . \qquad (5)$$

Weiterhin kann aus Fig. 16 entnommen werden, dass die Differenz aus geschätztem Störer und dem Eingangssignal das gefilterte Signal ergibt:

$$\vec{y}^A = \vec{w}^A - \vec{w}^s \; , \qquad (6)$$

oder

$$\vec{y}^A = \vec{w}^A - W_c^A \left( W_c^A \right)^{\#} \vec{w}^A = \vec{w}^A \left( E - W_c^A \left( W_c^A \right)^{\#} \right), \qquad (7)$$

Wobei E eine Einheitsmatrix repräsentiert. Eine alternative Realisierung der vorliegenden Erfindung wäre ein Filter, das auf Basis der Kenntnis der in dem Differenzsignal vorkommenden Frequenzen ein Notch-Filter realisiert, das in den Pfad des Nutzsignals geschaltet wird und die Frequenzen des Differenzsignals dämpft.

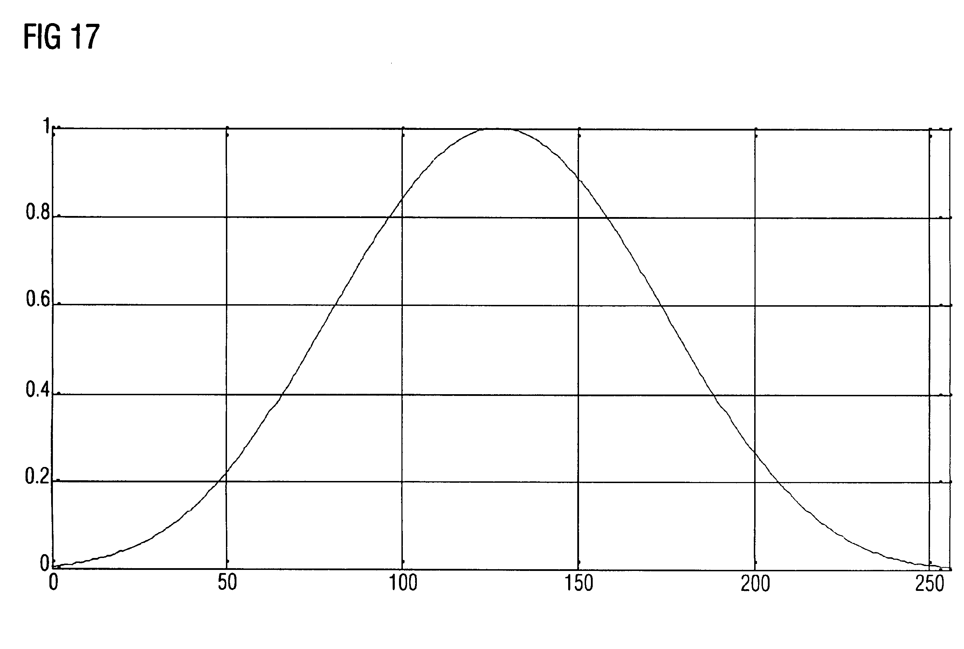

Da nachfolgend Untersuchungen im Frequenzbereich stattfinden, werden die Eingangssignale mittels der Fourier-Transformation in den Frequenzbereich transformiert. Durch die Blockbildung entstehen im Frequenzbereich unerwünschte Nebeneffekte. Eine Blockbildung ist gleichzusetze mit einer Multiplikation eines rechteckförmigen Impulses, der aus einem Empfangssignal gerade den betrachteten Block ausblendet mit dem Empfangssignal selbst. Wird nun auf diesen Block die Fourier-Transformation angewendet, so erhält man im Frequenzbereich eine Faltung des Fourier-transformierten Rechteckimpulses (Sinc-Funktion) mit dem eigentlichen Spektrum der Folge von Empfangssignalabtastwerten. Um die ungünstigen Effekte die durch die Faltung mit der Sinc-Funktion im Frequenzbereich hervorgerufen werden zu vermindern, wird der Block aus Empfangssignalabtastwerten im Zeitbereich mit einer Fensterfunktion multipliziert, die ein schmäleres Spektrum aufweist als die Sinc-Funktion. In der Realisierung des Ausführungsbeispiels wird hierzu eine Kaiser-Bessel-Funktion verwendet. In Fig. 17 ist der Signalverlauf eines Kaiser-Bessel-Fensters beispielhaft dargestellt. Die Multiplikation der Signalblöcke mit der Fensterfunktion kann wahlweise auch vor der adaptiven Filterung erfolgen.

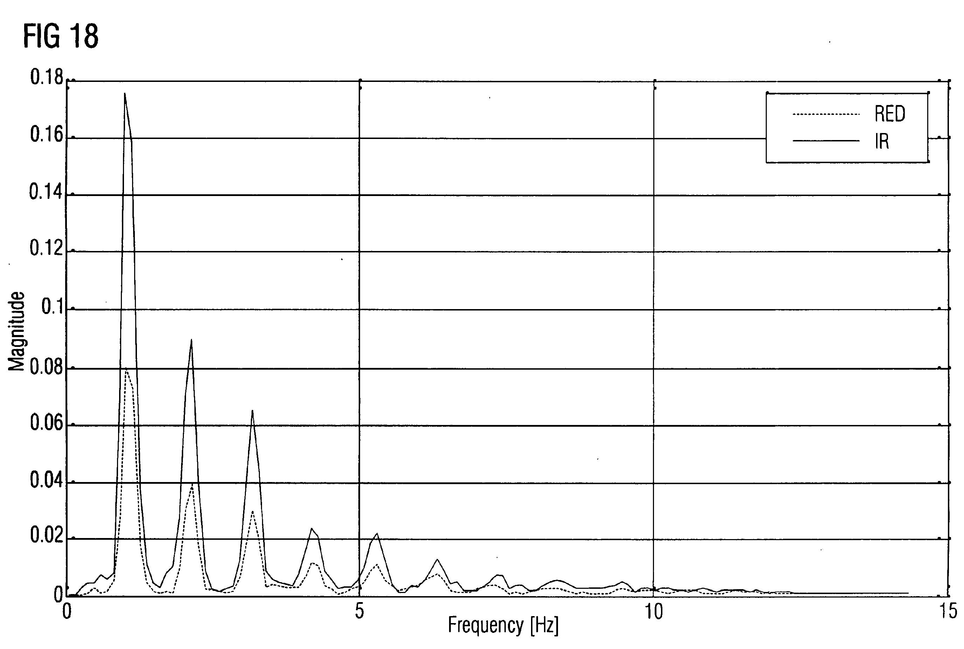

Zur weiteren Signalverarbeitung werden nun die beiden Nutzsignale normiert. Nachfolgend erfolgt die Fourier-Transformation. Nach der Fourier-Transformation können die Spektren in verschiedenen Ansichten dargestellt werden, wie z.B. deren Verlauf über die Zeit oder über der Frequenz. Fig. 18 zeigt zwei beispielhafte Spektren der normierten Signale aus den Hellsendekanälen Rot und Infrarot. Die Spektren zeigen Signale bei guten Bedingungen, d.h. mit verhältnismäßig geringer Störung. Nach der Fourier-Transformation 335 erfolgt in einem nächsten Signalverarbeitungsschritt das erfindungsgemäße Bestimmen des Referenzspektrums 338 und die Anwendung der erfindungsgemäßen spektralen Maske 340, zur Bestimmung der Herzfrequenz.

Aus den beiden Spektren, die aus den beiden Hellsendekanälen bestimmt wurden, wird erfindungsgemäß ein Referenzspektrum bestimmt. In diesem Ausführungsbeispiel liegen nach der Fouriertransformation zwei Spektren vor, deren Zeitsignale ohne Einfluss von Störungen perfekt miteinander korreliert und deren Spektren linear abhängig wären. Die Spektren werden hier als Vektoren beschrieben, und im ungestörten Falle ließe sich ein Spektrum als Linearkombination des anderen ausdrücken:

$$\vec{\gamma}_r \overset{!}{=} \rho \vec{\gamma}_i \qquad\qquad . \qquad (8)$$

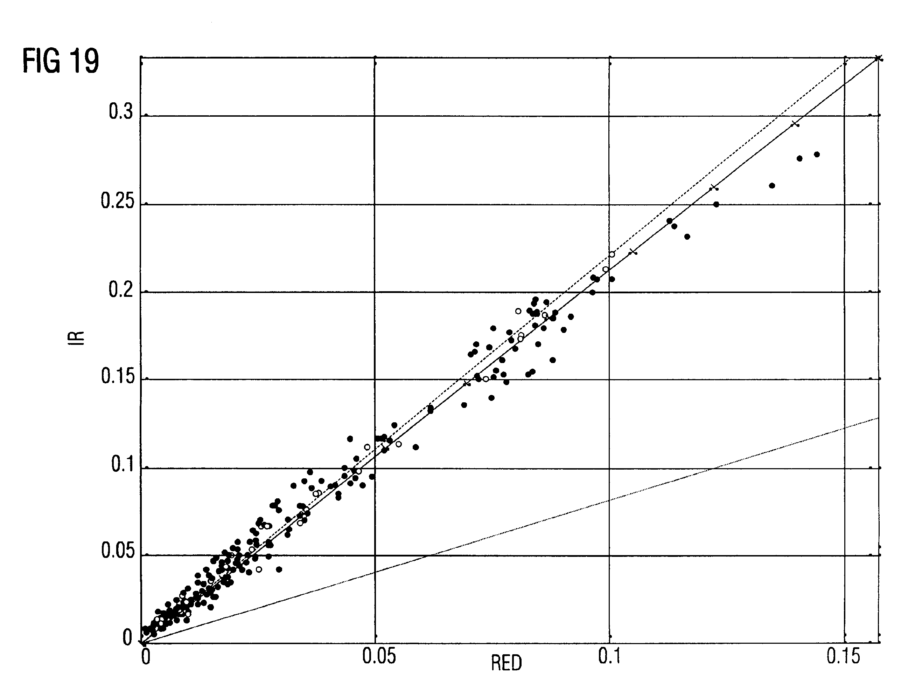

$\vec{\gamma}_r$ entspricht dabei dem Rot-Hellsendekanal, $\rho$ entspricht dem Linearkoeffizienten und $\vec{\gamma}_i$ entspricht dem Infrarot-Hell-sendekanal. Da beide Spektren gestört sind, ist das so entstandene Gleichungssystem überbestimmt und nicht lösbar. Wäre die Gleichung lösbar, würden also keine Störungen vorliegen, dann wäre der Skalar $\rho$ schon das Verhältnis, mit Hilfe dessen über die Kalibrierungsfunktion der gewünschte $SpO_2$-Wert bestimmt werden könnte. Da Gleichung (8) nicht lösbar ist, wird nun nach einem $\rho$ gesucht, das erfindungsgemäß am besten in die Gleichung passt. Dieses Problem kann veranschaulicht werden, indem die beiden Spektren in einem x-y-Plot aufgetragen werden. Fig. 19 veranschaulicht einen solchen x-y-Plot. Aufgrund von Störungen sind die Spektren unterschiedlich. Wären die Spektren identisch, würden die Punkte in Fig. 19 eine perfekte Ursprungsgerade ergeben. Da die Spektren allerdings gestört sind, bilden die in Fig. 19 aufgetragenen Punkte eine Wolke, durch die sich mehrere Geraden legen lassen.

In Fig. 19 ist ferner eine Gerade 1900 eingezeichnet, die den Wertebereich der vorkommenden Steigungen nach unten begrenzt. Die Gerade 1900 entspricht dabei einer Blutsauerstoffsättigung von 70%, die Gerade 1910 entspricht einer Blutsauerstoffsättigung von 100%. Die Gerade 1920 ist die Gerade, die mit dem erfindungsgemäßen Verfahren bestimmt wurde, die Bestimmung ihrer Steigung wird im Folgenden im Detail beschrieben. Gesucht wird eine Linearkombination mit einem Linearkoeffizienten $\rho$, der am besten die Gleichung (8) erfüllt. Gleichung (8) wird um zwei Störvektoren $\vec{i}_r$ und $\vec{i}_i$ erweitert, da aufgrund von Störungen, wie Bewegungsartefakten oder weißem Rauschen, zwei unterschiedliche Störsignale jedem Kanal additiv überlagert wurden. Die Spektren können jeweils in einen reinen stö-rungsfreien Pulssignalanteil ($\vec{\gamma}_{r,p},\vec{\gamma}_{i,p}$) und einen additiven Störanteil ($\vec{i}_{r,p},\vec{i}_{i,p}$) zerlegt werden,

$$\vec{\gamma}_r = \vec{\gamma}_{r,p} + \vec{i}_r \quad \text{und} \quad \vec{\gamma}_i = \vec{\gamma}_{i,p} + \vec{i}_i \qquad . \qquad (9)$$

Das über bestimmte Gleichungssystem lautet dann:

$$\vec{i}_r + \vec{\gamma}_{r,p} \overset{!}{=} \rho(\vec{\gamma}_{i,p} + \vec{i}_i) \qquad . \qquad (10)$$

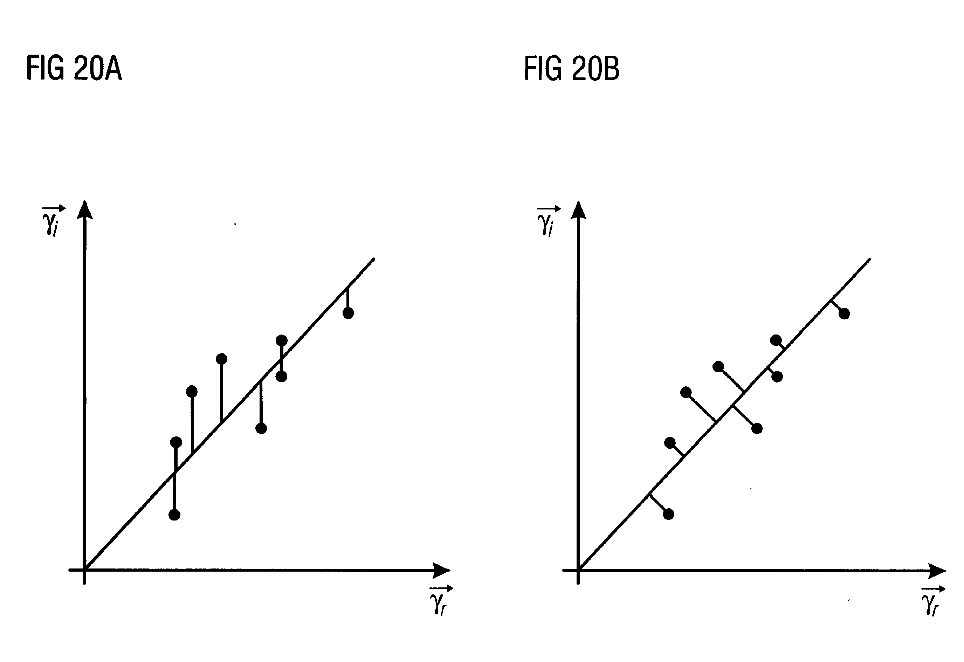

Das Problem lässt sich mit der Methode der kleinsten Quadrate (Least-Squares Fit) nicht lösen, da dies nur dann möglich wäre, wenn nur eines der beiden Signale mit Störungen überlagert worden wäre. Zum Vergleich wird in Fig. 20a das Least-Squares Fit-Verfahren dargestellt, das eine Minimierung einer vertikalen Distanz entspricht, und in Fig. 20b das Total-Least-Squares Fit-Verfahren, das der Minimierung der tatsächlichen Distanz zur zu bestimmenden Gerade entspricht. In beiden Fällen wird versucht, die Summe der Quadrate der eingezeichneten Distanzen zwischen den jeweiligen Punkten und einer Geraden zu minimieren, wobei weiter vom Ursprung liegende Punkte stärker gewichtet werden, was auch im vorliegenden Ausführungsbeispiel gewünscht ist. Koeffizienten, wie sie beispielsweise der Grundwelle eines Pulssignals entsprechen, werden höher gewertet als Koeffizienten von den verhältnismäßig schwächeren Oberwellen. Die Steigung der Geraden ist in beiden Fällen das Verhältnis $\rho$, allerdings ist das Total-Least-Squares Fit-Verfahren eine bessere Näherung, da nur hier die tatsächliche Distanz zu den Punkten berücksichtigt wird. Zur Lösung des Problems nach Gleichung (10) wird eine Singulärwertzerlegung (Englisch SVD = Singular Value Decomposition) herangezogen, mit der sich ein Total-Least-Square Fit-Verfahren vollziehen lässt. Die Singulärwertzerlegung basiert auf der Zerlegung einer beliebigen Matrix A in drei Matrizen:

$$A = U \sum V^+ \qquad . \qquad (11)$$

Das Produkt der drei Matrizen der Singulärwertzerlegung ergibt wieder die Matrix A. Für das hier vorgestellte Ausführungsbeispiel ist speziell die Matrix $\Sigma$ von Bedeutung. Die diagonalen Elemente der Matrix $\Sigma$ werden Singulärwerte genannt. Zur weiteren Untersuchung wird eine Matrix A aus den zwei Spaltenvektoren zusammengesetzt, die jeweils das Spektrum der ungestörten Signale mit ihren additiven Störern repräsentieren:

$$A = \begin{bmatrix} \vec{\gamma}_r & \vec{\gamma}_i \end{bmatrix} \in \mathbf{C}^{128 x 2} \qquad . \qquad (12)$$

Der Rang dieser Matrix ist mit hoher Wahrscheinlichkeit gleich 2, da es aufgrund der additiven Störer absolut unwahrscheinlich ist, dass die Spaltenvektoren linear abhängig sind. Mittels der Singulärwertzerlegung lässt sich zum einen der Rang der Matrix ermitteln, es lässt sich aber auch untersuchen, wie nah eine Matrix am niedrigern Rang ist. Es kann zur Matrix A eine möglichst ähnliche Matrix gefunden werden, deren Rang niedriger als der der Matrix A ist. Ließe sich der Rang der Matrix A auf 1 reduzieren, so würden die Spaltenvektoren linear abhängig werden, und die Spektren wären bis auf den Faktor $\rho$ gleich. Eines der so entstandenen Spektren könnte dann erfindungsgemäß zur weiteren Berechnung genommen werden, da es fortan die Information beider Kanäle tragen würde.
Führt man für A eine Singularwertzerlegung durch, so erhält man für $\Sigma$ eine Matrix der Form:

$$\Sigma = \begin{bmatrix} \Sigma_1 & 0 \\ 0 & \Sigma_2 \end{bmatrix} \qquad . \qquad (13)$$

Der Rang einer Matrix A entspricht der Anzahl aller von Null verschiedenen Singulärwerte. Da der Rang der Matrix A gleich 2 ist, sind beide Singulärwerte größer Null, ferner

$$\Sigma_1 \geq \Sigma_2 > 0 \qquad . \qquad (14)$$

Das Element $\Sigma_2$ verrät, wie nahe die Matrix $\Sigma$ am nächstniedrigeren Rang ist, im vorliegenden Ausführungsbeispiel also, wie nahe die Matrix $\Sigma$ am Rang 1 ist. Es folgt daraus, dass sich die Größe von $\Sigma_2$ nach der Intensität der überlagerten Störungen richtet, die den beiden Spektren überlagert sind und die voneinander unabhängig sind. Der Rang der Matrix A kann nun auf 1 reduziert werden, indem $\Sigma_2$ gleich Null gesetzt wird. Unabhängige Störungen, die den beiden Spektren überlagert sind, können auf diese Weise stark reduziert werden. Abhängige Störungen, also Störungen, die in beiden Signalen gleich sind oder linear voneinander abhängig sind, verbleiben zunächst in den beiden Spektren. Die Rekonstruktion der Matrix $\tilde{A}$ ergibt eine neue Matrix mit Rang 1:

$$\tilde{A} = U \begin{bmatrix} \Sigma_1 & 0 \\ 0 & 0 \end{bmatrix} V^+ \qquad . \qquad (15)$$

Das Resultat ist eine Matrix deren Spaltenvektoren linear abhängig sind, und die sich aus der Anwendung des Total-Least-Squares-Fit-Verfahrens auf die beiden mit Störungen überlagerten Spaltenvektoren der Matrix A ergibt. Die beiden Spaltenvektoren sind komplexe Spektren und stellen zugleich die beste Näherung an die mit Störungen überlagerten Spektren dar. Erfindungsgemäß könnten die beiden Spektren auch nach einem anderen Optimierungskriterium, wie beispielsweise dem Least-Squares Fit-Verfahren bestimmt werden. Aus den linear abhängigen Spektren der Matrix

$\tilde{A}$, wurden auf die beschriebene Art und Weise die unabhängigen Störungen, wie beispielsweise adaptives weißes Rauschen, entfernt, die Matrix $\tilde{A}$ ergibt sich nun als

$$\widetilde{A} = \begin{bmatrix} \bar{\gamma}_{r,f} & \bar{\gamma}_{i,f} \end{bmatrix} \in \mathbf{C}^{128x2} \quad . \qquad\qquad (16)$$

Mit den so bestimmten Spaltenvektoren ergibt sich ein lösbares Gleichungssystem für das Verhältnis $\rho$ zur Berechnung der Sauerstoffsättigung,

$$\bar{\gamma}_{r,f} = \rho\,\bar{\gamma}_{i,f} \qquad . \qquad\qquad (17)$$

Das Verhältnis $\rho$ wird an dieser Stelle jedoch noch nicht verwendet, denn wenn sich eine linear abhängige Störung hoher Amplitude den beiden Spektren identisch überlagert hat, so kann diese mit dem hier vorgeschlagenen Verfahren nicht reduziert werden. Um nun diese Art von Störung aus dem bestimmten Spektrum herauszufiltern, wird der Schritt der spektralen Maske 340 zur Bestimmung einer biologischen Größe wie beispielsweise der Herzfrequenz herangezogen. Als Berechnungsgrundlage benötigt der Schritt der spektralen Maske 340 das Referenzspektrum, das repräsentativ für beide Kanäle ist. Da die beiden Spektren aus Gleichung 16 linear voneinander abhängig sind, repräsentiert jedes der beiden Spektren beide Hellsendekanäle.

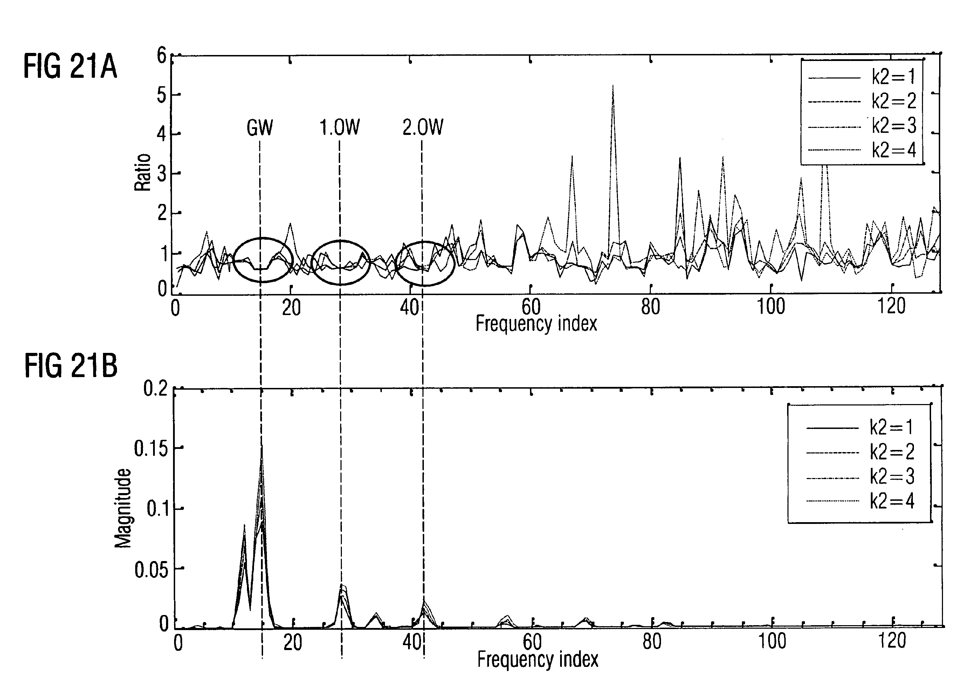

Die Funktion der spektralen Maske 340 ist, die Fourier-Koeffizienten des Puls-Signals im Spektrum zu suchen, um alle anderen Koeffizienten auf Null zu setzen, und so Frequenzanteile, die nicht zum Pulssignal gehören, von der weiteren Analyse auszuschließen. Alle anderen Koeffizienten tragen zu einer falschen Bestimmung des Linearkoeffizienten $\rho$ bei. Zur Veranschaulichung wird zunächst punktweise der Linearkoeffizient der beiden gestörten Spektren (Rot und Infrarot) gebildet, so dass für jede einzelne Spektrallinie ein Linearkoeffizient $\rho$ entsteht, wie es in Fig. 21a) und Fig. 21b) dargestellt ist. Fig. 21a veranschaulicht den beispielhaften Verlauf des Linearkoeffizienten p (in der Figur als Ratio bezeichnet) aus zwei gestörten Spektren der Signalverläufe der Hellsendekanäle, Fig. 21b zeigt dazu den von Störung bereinigten Verlauf eines Referenzspektrums. Beide spektralen Verläufe sind zu jeweils vier verschiedenen Zeitpunkten aufgetragen, k2=1..4. Vergleicht man nun den Linearkoeffizienten der beiden Spektren aus Fig. 21a mit dem Referenzspektrum über mehrere Zeitfenster, so wird deutlich, dass der Linearkoeffizient nur über den Frequenzkomponenten des Pulssignals korrekt ist, und für all diese Frequenzen gleich ist. Dies führt zunächst zu keinem nennenswerten Fehler in der Berechnung des Linearkoeffizienten mit der Singularwertzerlegung, denn bei einem Total-Squares Fit-Verfahren wird der Linearkoeffizient bei höheren Amplituden stärker gewichtet als bei niedreren Amplituden.

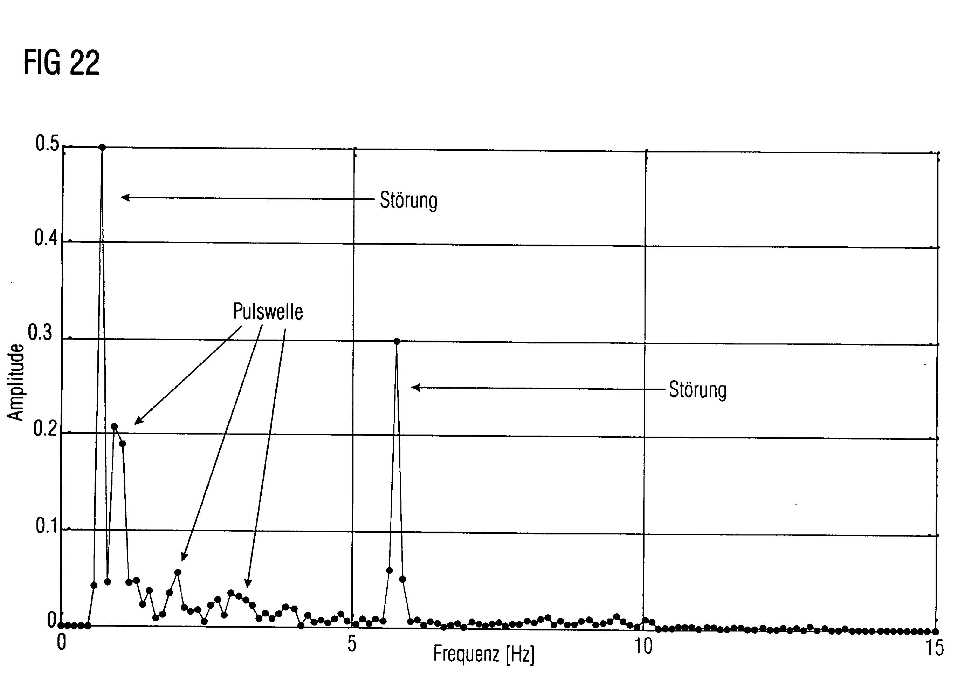

Problematisch wird es dann, wenn die Amplituden der Störungen größer als die Pulswelle werden, wie es in Fig. 22 schematisch dargestellt ist. Fig. 22 zeigt beispielhaft ein Spektrum eines Signals, das durch Störsignale gestört wird, deren Amplituden größer sind als die Amplituden der eigentlichen Pulswelle. Der Quotient zweier Spektren ist bei den Frequenzen eines Störers undefiniert und hat keinen Bezug zur Blutsauerstoffsättigung eines Probanden. Ohne die spektrale Maske 340 würden dominante Störungen wie in Fig. 22 dargestellt, zu einem falschen Blutsauerstoffsättigungswert führen. Erfahrungsgemäß beträgt der Linearkoeffizient bei solch dominanten Störern im Spektrum den Wert 1, denn die Störung ist meistens auf Lichtquellen zurückzuführen, die in beiden Spektren mit der gleichen Amplitude vorzufinden sind. Ein Linearkoeffizient gleich 1 bedeutet je nach Kalibrierungskurve etwa 80% Blutsauerstoffsättigung. Ohne der erfindungsgemäßen hier vorgestellten spektralen Maske, würden im Spektrum dominant auftretende Störungen zu einem unzuverlässigen oder falschen $SpO_2$-Wert von etwa 80% führen, ohne dabei die tatsächliche Sättigung relevant zu berücksichtigen.

Der Algorithmus der spektralen Maske ist in der Lage Frequenzkomponenten der Pulswelle von denen der Störer zu unterscheiden. Der Algorithmus liefert eine binäre Maske mit den Elementen {0,1} liefern, mit denen das Spektrum punktweise multipliziert werden kann, um so die nicht zum Pulssignal gehörenden Fourierkoeffizienten zu unterdrücken.

Die spektrale Maske verfügt über einen Algorithmus der harmonischen Beziehung. Die Methode der harmonischen Beziehung basiert auf Erkenntnissen auf Untersuchen zahlreicher Pulssignale auf ihre spektralen Eigenschaften hin. Die fundamentale Erkenntnis ist die harmonische Beziehung der drei relevanten Frequenzen $f_g$ der Grundwelle, $f_{o1}$ der ersten Oberwelle und $f_{o2}$ der zweiten Oberwelle. Dabei ist weiterhin bekannt, dass die zweite Oberwelle bei der doppelten Frequenz der Grundquelle liegt, und dass die dritte Oberwelle bei der dreifachen Frequenz der Grundwelle liegt. Basierend auf dieser Beziehung kann nun eine Maske erstellt werden, die im Frequenzbereich jeweils die Frequenzanteile der doppelten und dreifachen Frequenz einer Grundfrequenz einblendet, das heißt, an diesen Stellen eine 1 aufweist, und alle anderen Frequenzen ausblendet, das heißt an diesen Stellen eine Null aufweist. Aus den verbleibenden Koeffizienten kann dann eine Summe gebildet werden, die der Grundfrequenz zugeordnet wird. Dieser Vorgang kann dann für alle möglichen denkbaren Herzfrequenzen, beispielsweise in einem Bereich von 30-300Hz wiederholt werden, und anschließend kann diejenige Frequenz selektiert werden, bei der die Summe maximiert wird.

Eine weitere Eigenschaft, die dabei berücksichtigt werden kann, ist dass die Amplituden der jeweiligen Oberwellen eine abklingende Charakteristik aufweisen. Dies bedeutet, dass die Amplitude bei der ersten Oberwelle oder bei der doppelten Frequenz der Grundwelle eine kleinere Amplitude aufweist, als die Grundwelle selbst. Bei der zweiten Oberwelle, die die dreifache Frequenz der Grundwelle aufweist, ist die Amplitude wiederum geringer als bei der ersten Oberwelle. Bei der Maximumsuche werden Werte nicht beachtet, für die die betreffende Bedingung des abklingenden Spektrums nicht erfüllt ist.

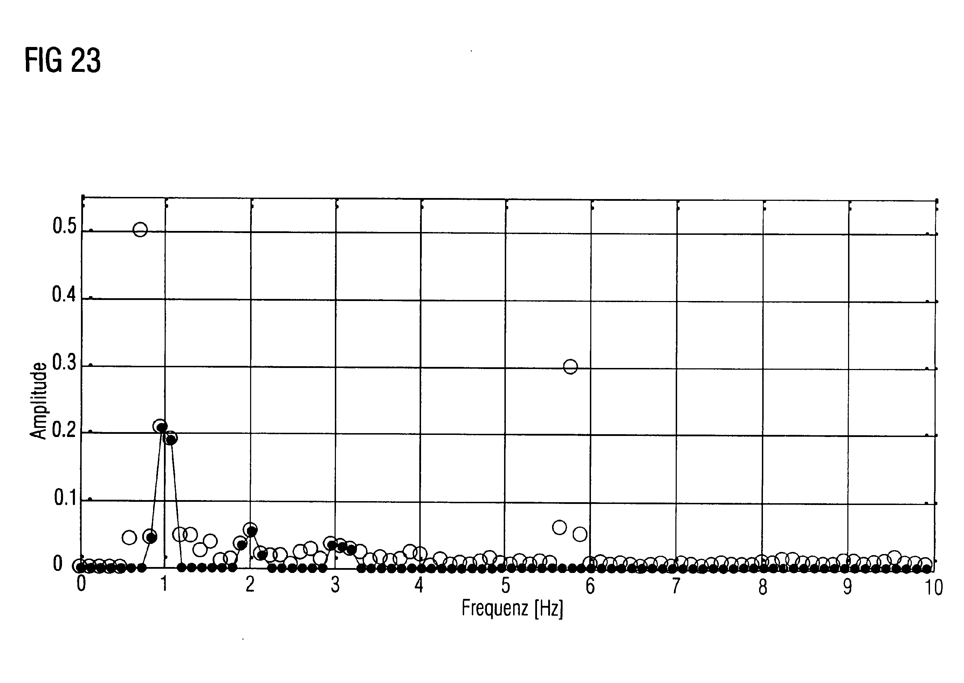

Fig. 23 zeigt ein Referenzspektrum (leere Punkte) und die nach der Anwendung der spektralen Maske ausgewählten Koeffizienten (volle Punkte). Es ist zu erkennen, dass die beiden Störer bei etwa 0,75 Herz und 5,75 Herz nach der Anwendung der spektralen Maske im neuen Spektrum nicht mehr vorzufinden sind.

Die Herzfrequenz kann jetzt über die Lage der spektralen Maske im Spektrum bestimmt werden. Im Ausführungsbeispiel der vorliegenden Erfindung nach Fig. 3, wird die Herzfrequenz dann am Ausgang 345 ausgegeben.

Wie bereits oben erwähnt, könnte man den Linearkoeffizienten $\rho$ schon aus der ersten Anwendung des Total-Least-Squares-Fit-Verfahrens berechnen, allerdings führen hier Störungen hoher Amplitude zu einem Fehler in der Messgröße. Nach der Multiplikation mit der spektralen Maske, werden nur die relevanten Frequenzkomponenten selektiert. Es wird nun erfindungsgemäß erneut das Total-Least-Squares-Fit-Verfahren mit der Singulärwertzerlegung auf die selektierten Frequenzkomponenten angewendet.

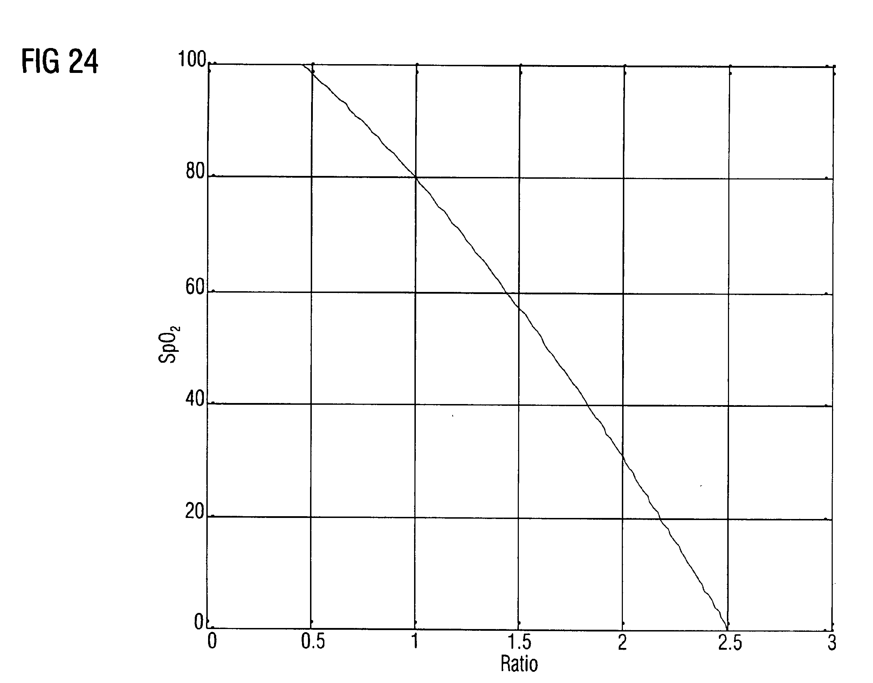

Hierbei werden nur die Frequenzkomponenten verwendet, die mit Hilfe der spektralen Maske bestimmt wurden. Über diese störungsbereinigten Spektren kann nun die Ursprungsgerade und deren Steigung bestimmt werden. Neben der Steigung der Ursprungsgeraden, kann aus der Matrixzerlegung des überbestimmten linearen Gleichungssystems auch ein Maß für die Zuverlässigkeit der bestimmten Steigung extrahiert werden. Die Varianz nach der Frobenius-Norm, die direkt aus der Matrixzerlegung gewonnen werden kann, gibt Aufschluss über die Ähnlichkeit der beiden Signale. Die Varianz wird als Indikator für übermäßige Störeinflüsse verwendet, die die Berechnung der Vitalparameter innerhalb der festgelegten Toleranz verhindert. Diese Varianz kann dann nach Fig. 3 am Ausgang 355 ausgegeben werden. Dem Complex Total Least Squares Fit-Verfahren ist nachgeschaltet eine Kalibrierungsfunktion 360. Die durch das Complex Total Least Squares Fit-Verfahren bestimmte Steigung der Ursprungsgeraden, die repräsentativ für den Blutsättigungswert des Probanden ist, wird an eine Kalibrierungsfunktion 360 weitergegeben. Die Kalibrierungsfunktion ordnet den erhaltenen Steigungswerten direkt $SpO_2$-Werte (Blutsättigungswerte) zu. Die jeweiligen $SpO_2$-Werte werden dann gemäß Fig. 3 am Ausgang 365 ausgegeben. Fig. 24 zeigt eine beispielhafte Kennlinie einer Kalibrierungsfunktion. Es ist zu erkennen, wie Quotienten (Ratio) Blutsättigungswerte ($SpO_2$-Werte) zugeordnet werden. Die Kennlinien der Kalibrierungsfunktion werden empirisch anhand von Referenzmessungen bestimmt.

Vorteil der hier vorliegenden Erfindung ist, dass die speziell auf das Anwendungsgebiet der Plethysmographie und Pulsoximetrie zugeschnittene spektrale Analyse, die Zuverlässigkeit der Plethysmogramme erheblich verbessert, sowie eine effektive Maßnahme zur Unterdrückung von Störungen beispielsweise durch elektromagnetische Felder (z. B. Hochfrequenzchirurgie) ermöglicht.

Ein weiterer Vorteil ist, dass durch den Einsatz der Singulärwertzerlegung zur Berechnung der $SpO_2$-Werte aus den komplexen Spektren, ebenfalls ein Zuverlässigkeitsmaß in Form einer Varianz extrahiert werden kann und zur Beurteilung der Ergebnisqualität herangezogen werden kann, bzw. dadurch eine Fehlfunktion zuverlässig detektiert werden kann.

Ein zusätzlicher Vorteil ist, dass mit der erfindungsgemäßen Vorrichtung zur Messung der Blutsauerstoffsättigung der und Herzfrequenz, auch bei niedriger arterieller Blutvolumenpulsation bei Bewegung des Patienten zuverlässig gemessen werden kann, was auf die durch das erfindungsgemäß Verfahren gewonnene Zuverlässigkeit zurückzuführen ist.

Generell lässt sich sagen, dass durch die vorliegende Erfindung die Behandlungsqualität eines Patienten insbesondere bei der intensivmedizinischen Versorgung und in Operationssälen erheblich verbessert werden kann. Durch die erhöhte Zuverlässigkeit und Robustheit des Verfahrens, können Fehldiagnosen, die auf störungsbehaftete Messungen bzw. auf unzuverlässige Messwerte zurückzuführen sind, erheblich reduziert werden.

Die erhöhte Zuverlässigkeit der Messung bedingt unmittelbar eine Steigerung der Behandlungsqualität eines Patienten. Damit ist ein Vorteil der vorliegenden Erfindung, dass durch die gesteigerte Zuverlässigkeit der Messwerte eines Pulsoximeters, insbesondere in kritischen Umgebungen, wie Operationssälen oder Intensivstationen, höhere Genesungschancen und effizientere Behandlungsmethoden ermöglicht werden.

**Bezugszeichenliste**

100    Vorrichtung zum Bestimmen des Spektralverhältnisses

EP 1 909 189 A2

| | |
|---|---|
| 110 | Rechner |
| 112 | erster Eingang des Rechners |
| 114 | zweiter Eingang des Rechners |
| 116 | erster Ausgang des Rechners |
| 118 | zweiter Ausgang des Rechners |
| 120 | Optimierer |
| 122 | erster Eingang des Optimierers |
| 124 | zweiter Eingang des Optimierers |
| 126 | Ausgang des Optimierers |
| 130 | Einrichtung zum Bereitstellen der Signale |
| 132 | erster Ausgang der Einrichtung zum Bereitstellen der Signale |
| 134 | zweiter Ausgang der Einrichtung zum Bereitstellen der Signale |
| 136 | erster Eingang der Einrichtung zum Bereitstellen der Signale |
| 138 | zweiter Eingang der Einrichtung zum Bereitstellen der Signale |
| 300 | Spreizspektrummodulation |
| 305 | LED-Treiber |
| 310 | Gewebe |
| 315 | Photoempfänger |
| 320 | Analog/Digital-Wandeleinrichtung |
| 325 | Spreizspektrumdemodulator |
| 330 | adaptives Filter |
| 335 | Fouriertransformation |
| 338 | Referenzspektrum |
| 340 | spektrale Maske |
| 345 | Herzfrequenz |
| 350 | Komplex Total Least Squares Fit |
| 355 | Varianzzuverlässigkeitsmaß |
| 360 | Kalibrierungsfunktion |
| 365 | $SpO_2$-Wert |
| 1900 | untere Schranke der Blutsauerstoffsättigung |
| 1910 | obere Schranke der Blutsauerstoffsättigung |
| 1920 | erfindungsgemäß bestimmte Gerade |

**Patentansprüche**

1. Vorrichtung (100) zum Bestimmen eines Spektralverhältnisses zwischen einem ersten Signal mit einem ersten Spektrum, das von einer biologischen Größe abhängt und einem zweiten Signal mit einem zweiten Spektrum, das von einer biologischen Größe abhängt, wobei das erste Signal und das zweite Signal eine Mehrzahl von Harmonischen eines periodischen Signals haben, mit folgenden Merkmalen:

   einem Rechner (110) zur Berechnung eines ersten Wellenverhältnisses zwischen einem Spektralwert des ersten Spektrums, der eine Frequenz einer Harmonischen der Mehrzahl von Harmonischen hat, und einem Spektralwert des zweiten Spektrums, der die Frequenz der Harmonischen hat;
   und zur Berechnung eines zweiten Wellenverhältnisses zwischen einem Spektralwert des ersten Spektrums, der eine Frequenz einer anderen Harmonischen der Mehrzahl von Harmonischen hat, und einem Spektralwert des zweiten Spektrums, der die Frequenz der anderen Harmonischen hat;
   einem Optimierer (120) zum Ermitteln des Spektralverhältnisses unter Verwendung des ersten und des zweiten Wellenverhältnisses, wobei der Optimierer ausgebildet ist, um das Spektralverhältnis so zu ermitteln, dass es sich von dem ersten Wellenverhältnis und dem zweiten Wellenverhältnis unterscheidet und ein Optimierungskriterium erfüllt.

2. Vorrichtung (100) gemäß Anspruch 1, wobei die Periode des periodischen Signals von der biologischen Größe unabhängig ist.

3. Vorrichtung gemäß einem der Ansprüche 1 oder 2, wobei das erste Signal und das zweite Signal auf unterschiedliche Weise von der biologischen Größe abhängen.

17

**4.** Vorrichtung (100) gemäß einem der Ansprüche 1 bis 3, wobei das erste und das zweite Signal Abhängigkeiten von der biologischen Größe in der Mehrzahl der Harmonischen aufweisen.

**5.** Vorrichtung (100) gemäß einem der Ansprüche 1 bis 4, bei der die beiden Signale auf optischen Signalen basieren, deren Wellenlängen so gewählt sind, dass die Dämpfung des einen optischen Signals von der biologischen Größe abhängt und die Dämpfung des anderen optischen Signals nicht von der biologischen Größe abhängt.

**6.** Vorrichtung (100) gemäß einem der Ansprüche 1 bis 5, wobei die biologische Größe eine Blutsauerstoffsättigung oder eine Herzfrequenz ist.

**7.** Vorrichtung (100) gemäß einem der Ansprüche 1 bis 6, wobei der Rechner (110) ausgebildet ist, um das erste Wellenverhältnis bei einer Grundwellenfrequenz des periodischen Signals und das zweite Wellenverhältnis bei einer Oberwellenfrequenz des periodischen Signals zu berechnen.

**8.** Vorrichtung (100) gemäß einem der Ansprüche 1 bis 7, wobei der Rechner (110) ausgebildet ist, um Wellenverhältnisse zwischen einer Mehrzahl von Spektralwerten des ersten und des zweiten Signals zu bilden und ferner der Optimierer (120) ausgebildet ist, um das Spektralverhältnis aus der Mehrzahl von Wellenverhältnissen zu bilden.

**9.** Vorrichtung (100) gemäß einem der Ansprüche 1 bis 8, wobei die Signale aus einer Durchleuchtung eines Gewebes eines Probanden entstammen.

**10.** Vorrichtung (100) gemäß einem der Ansprüche 1 bis 9, wobei ein Signal auf einem Infrarotlicht-Empfangssignal und ein Signal auf einem Rotlicht-Empfangssignal beruht.

**11.** Vorrichtung (100) gemäß einem der Ansprüche 1 bis 10, wobei der Optimierer (120) ausgebildet ist, um ein kumuliertes Abstandsmaß zwischen dem Spektralverhältnis und den Wellenverhältnissen zu optimieren.

**12.** Vorrichtung (100) gemäß einem der Ansprüche 1 bis 11, wobei der Rechner (110) ausgebildet ist, um eine Mehrzahl der Spektralwerte der zwei Spektren als Tupel einer Frequenz an den Optimierer (120) weiterzugeben, und der Optimierer (120) ausgebildet ist, um aus der Mehrzahl von Tupel eine Regressionsgerade zu bestimmen und auszugeben, wobei der horizontale, der vertikale oder der senkrechte, der lineare oder der quadratische Abstand minimiert wird.

**13.** Vorrichtung (100) gemäß einem der Ansprüche 1 bis 12, wobei der Optimierer (120) ausgebildet ist, um die Regressionsgerade nach dem Least-Squares-Fit oder dem Total-Squares-Fit-Verfahren zu bestimmen.

**14.** Vorrichtung (100) gemäß einem der Ansprüche 1 bis 13, wobei der Optimierer (120) ausgebildet ist, um die Regressionsgerade mit Hilfe einer Singulärwertzerlegung einer Matrix zu bestimmen, wobei die beiden Signale die Spalten der Matrix bilden, und der betragsmäßig kleinere Singulärwert der Singulärwertzerlegung zur Linearisierung des Gleichungssystems zu Null gesetzt wird.

**15.** Vorrichtung (100) gemäß einem der Ansprüche 1 bis 14, die ferner eine Einrichtung (130) zum Bereitstellen des ersten und des zweiten Signals aufweist, und die ferner ausgebildet ist, um basierend auf Vorkenntnissen über den Spektralverlauf eines Signalanteils, Frequenzanteile zu vermindern.

**16.** Vorrichtung (100) gemäß Anspruch 15, wobei die Einrichtung (130) zum Bereitstellen des ersten und des zweiten Signals ausgebildet ist, um basierend auf bekannten Intensitätsverhältnissen zwischen den Harmonischen, andere Spektralanteile zu vermindern.

**17.** Vorrichtung (100) gemäß einem der Ansprüche 15 oder 16, wobei die Einrichtung (130) zum Bereitstellen des ersten und des zweiten Signals ausgebildet ist, um nur Spektralanteile der Grundwelle, der ersten Oberwelle, und der zweiten Oberwelle des periodischen Signals bereitzustellen.

**18.** Vorrichtung (100) gemäß einem der Ansprüche 15 bis 17, wobei die Einrichtung (130) zum Bereitstellen des ersten und des zweiten Signals ausgebildet ist, um von Signalen nur diejenigen Signalanteile bereitzustellen, die von ihren Frequenz- und Amplitudenverhältnissen einem Pulssignals eines Lebewesens entsprechen.

**19.** Vorrichtung (100) gemäß einem der Ansprüche 15 bis 18, wobei die Einrichtung (130) zum Bereitstellen des ersten und des zweiten Signals ausgebildet ist, um basierend auf einem Referenzspektrum, Pulssignalanteile eines Lebewesens zu bestimmen und aus den Signalen andere Frequenzen auszublenden.

20. Vorrichtung (100) gemäß einem der Ansprüche 1 bis 19, wobei der Rechner (110) ausgebildet ist, die Signale an den Optimierer (120) weiterzuleiten, und der Optimierer (120) ausgebildet ist, um basierend auf der Spektren der Signale ein Referenzspektrum zu bestimmen, für das ein Abstandsmaß zu den Spektren der Signale optimiert ist.

21. Vorrichtung (100) gemäß einem der Ansprüche 1 bis 20, wobei die zwei Signale aus einem Plethysmogramm stammen.

22. Vorrichtung (100) gemäß einem der Ansprüche 1 bis 21, die eine zweite Einrichtung zum Bereitstellen eines ersten zeitdiskreten Referenzsignals, das einen ersten Störanteil aufweist, und eines zweiten zeitdiskreten Referenzsignals, das einen zweiten Störanteil aufweist, wobei der zweite Störanteil zum ersten Störanteil phasenverschoben ist; und einer Subtrahiereinrichtung zum Erzeugen eines Differenzsignals aus den zwei Referenzsignalen, wobei das Differenzsignal eine Frequenzkomponente aufweist, die durch den ersten und den zweiten Störanteil verursacht ist, und
einer Einrichtung zum Manipulieren der zwei Signale, basierend auf dem Differenzsignal derart, dass in einem manipulierten Signal die Frequenzkomponente reduziert ist; und
das manipulierte zeitdiskrete Signal dem Rechner zur Verfügung gestellt wird.

23. Vorrichtung (100) gemäß einem der Ansprüche 1 bis 22, die eine erste Einrichtung zum Bereitstellen des zeitdiskreten Signals aufweist, die angepasst ist, um ein sich wiederholendes optisches Signal abzutasten, das einer Hellzeitdauer entspricht, in der eine Sendelichtquelle einen Einzustand annimmt, und eine zweite Einrichtung zum Bereitstellen eines zeitdiskreten Referenzsignals aufweist, die angepasst ist, um zwei optische Signale abzutasten, die Dunkelzeitdauern entsprechen, in denen keine Sendelichtquelle einen Einzustand annimmt.

24. Vorrichtung (100) gemäß einem der Ansprüche 1 bis 23, die eine erste Einrichtung zum Bereitstellen eines zeitdiskreten Signals aufweist, die ausgebildet ist, um ein sich wiederholendes optisches Signal zu verarbeiten, wobei das optische Signal Sequenzen aufweist und eine Sequenz wenigstens zwei Hellzeitdauern aufweist, in denen eine Sendelichtquelle einen Einzustand annimmt, und wenigstens eine Dunkelzeitdauer aufweist, in der keine Sendelichtquelle den Einzustand einnimmt, und die wenigstens zwei Hellzeitdauern in einer Sequenz unregelmäßig angeordnet sind, und die Einrichtung zum Bereitstellen des zeitdiskreten Signals ferner ausgebildet ist, um basierend auf der Information über die Anordnung der Hellzeitdauern in der Sequenz, das zeitdiskrete Signal entsprechend eines Hellsendesignals zur Verfügung zu stellen.

25. Vorrichtung (100) gemäß Anspruch 24, wobei der Sequenz eines optischen Signals ein Takt zugrunde liegt, gemäß dem die Hell- und Dunkelzeitdauern auftreten.

26. Vorrichtung (100) gemäß Anspruch 25, wobei die Einrichtungen zum Bereitstellen der zeitdiskreten Signale ausgebildet sind, um bei einem Takt größer als 800Hz zu arbeiten.

27. Vorrichtung (100) gemäß einem der Ansprüche 1 bis 26, wobei die Signale einer Spreizspektrummodulation entstammen.

28. Verfahren zum Bestimmen eines Spektralverhältnisses zwischen einem ersten Signal mit einem ersten Spektrum, das von einer biologischen Größe abhängt und einem zweiten Signal mit einem zweiten Spektrum, das von einer biologischen Größe abhängt, wobei das erste Signal und das zweite Signal eine Mehrzahl von Harmonischen eines periodischen Signals haben, mit folgenden Schritten:

Berechnen eines ersten Wellenverhältnisses zwischen einem Spektralwert des ersten Spektrums, der eine Frequenz einer Harmonischen der Mehrzahl von Harmonischen hat und einem Spektralwert des zweiten Spektrums, der die Frequenz der Harmonischen hat;
Berechnen eines zweiten Wellenverhältnisses zwischen einem Spektralwert des ersten Spektrums, der eine Frequenz einer anderen Harmonischen der Mehrzahl der Harmonischen hat und einem Spektralwert des zweiten Spektrums, der die Frequenz der anderen Harmonischen hat;
Ermitteln des Spektralverhältnisses unter Verwendung des ersten und des zweiten Wellenverhältnisses, wobei das Spektralverhältnis so ermittelt wird, dass es sich von dem ersten und dem zweiten Wellenverhältnis unterscheidet und ein Optimierungskriterium erfüllt.

**FIG 1A**

**FIG 1B**

FIG 2A

FIG 2B

**FIG 3**

```
┌─────────────┐        ┌─────────────┐
│     [2]     │ ◄───── │     [1]      │
│ LED-Treiber │        │ Spreizspektrum-│
│             │        │ modulation   │
└──────┬──────┘        └─────────────┘
       │ │              \_305         \_300
       ▼ ▼
┌─────────────┐
│   Gewerbe   │
└──────┬──────┘
       │ │   \_310
       ▼ ▼
┌─────────────┐  ┌─────────────┐  ┌─────────────┐  ┌─────────────┐
│     [3]     │→ │     [4]     │→ │     [5]     │→ │     [6]     │
│Photoempfänger│  │ A/D-Wandler │  │Spreizspektrum-│ │  Adaptive   │
│             │  │             │  │demodulation │  │  Filterung  │
└─────────────┘  └─────────────┘  └─────────────┘  └──────┬──────┘
  \_315            \_320            \_325            \_330 │
                                                          ▼
┌─────────────┐  ┌─────────────┐  ┌─────────────┐  ┌─────────────┐
│    [10]     │◄ │     [9]     │◄ │     [8]     │◄ │     [7]     │
│Kalibrierungs-│  │Complex Total│  │  Spektrale  │  │  Fourier-   │
│  funktion   │  │Least Squares│  │    Maske    │  │Transformation│
│             │  │     Fit     │  │             │  │             │
└──────┬──────┘  └──────┬──────┘  └──────┬──────┘  └─────────────┘
       │  \_365  \_360  │   \_355  \_350  │   \_345  \_335
       ▼                ▼                 ▼
     SpO₂            Varianz         Herzfrequenz
```

EP 1 909 189 A2

22

EP 1 909 189 A2

FIG 4

$|\mathbf{I}(f)|$

$f_B$

$f$

FIG 5

$|\mathbf{I}_A(f)|$

$f_{AM}$

$f$

# FIG 6

Chipfolgen

# FIG 7

Betrag der Fourierkoeffizienten

EP 1 909 189 A2

FIG 8

$|\underline{I}_C(f)|$

f

# FIG 9

a) Amplitude / f

b) Amplitude / f

c) Amplitude / f

Störung 2

Störung 1

Moduliertes Pulssignal

d) Amplitude / f

Verbleibende gespiegelte Frequenzen

FIG 10

Primary extracted sensor signals

FIG 11

Primary extracted sensor signals

EP 1 909 189 A2

FIG 12

response of extraction filters

response of extraction filters

EP 1 909 189 A2

FIG 13

correlated sensor signals

correlated sensor signals

RF interference

RED
IR
RF interference

Amplitude

Time in sec

Time in sec

EP 1 909 189 A2

FIG 14

FIG 15A

Eingangssignal
DC-Anteil

FIG 15B

DC-Anteil

FIG 16

# FIG 17

EP 1 909 189 A2

FIG 18

EP 1 909 189 A2

FIG 19

EP 1 909 189 A2

FIG 20B

FIG 20A

FIG 21A

FIG 21B

# FIG 22

EP 1 909 189 A2

FIG 23

FIG 24

EP 1 909 189 A2

## FIG 25

Photosensor

Verarbeitung

Anzeige

## FIG 26

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1374764 A1
- WO 2002054950 A
- EP 208201 A2
- EP 341059 A3
- EP 314331 B1
- EP 1254628 A1
- US 5503144 A
- US 6714803 B